# EUROPEAN PATENT APPLICATION

(11) **EP 2 458 019 A2**
(43) Date of publication of application: **30.05.2012**
(21) Application number: 11194894.9
(22) Date of filing: 25.05.2006
(51) Int. Cl.: C12Q 1/70

(54) **Methods and compositions for determining resistance of HIV-1 to protease inhibitors**

(30) Priority: 27.05.2005 US 68533605 P; 16.12.2005 US 75089205 P
(62) Divisional of application: 06771316.4
(71) Applicant: Monogram BioSciences, Inc., South San Francisco, CA 94080 (US)
(72) Inventor: Parkin, Neil T., Belmont, CA 94002 (US)
(74) Representative: Jones Day

(57) **Abstract**

This invention relates to methods for determining resistance of HIV-1 viruses to protease inhibitors (PIs) based on the viral genotypes. The methods generally comprise detecting, in a gene encoding protease of the HIV-1, the presence of a mutation in at least one of codon 22, 69, 74, or 83 alone or in combination with one or more mutations at certain other codons, or, in a gene encoding gag of the HIV-1, the presence of a mutation in at least one of codon 418 or 482 alone or in combination with one or more mutations at certain other codons. Combinations of mutations associated with resistance to PIs are also disclosed.

## Description

### 1. FIELD OF INVENTION

This invention relates, in part, to methods and compositions for determining resistance of a human immunodeficiency virus ("HIV") to protease inhibitors ("PIs"), *e.g.,* amprenavir ("AMP"), indinavir ("IDV"), nelfinavir ("NFV"), ritonavir ("RTV"), saquinavir ("SQV"), lopinavir ("LPV") and/or atazanavir ("ATV"), by detecting the presence of a mutation or combinations of mutations in the gene encoding HIV protease or gag that are associated with resistance to the PIs.

### 2. BACKGROUND OF THE INVENTION

More than 60 million people have been infected with the human immunodeficiency virus ("HIV"), the causative agent of acquired immune deficiency syndrome ("AIDS"), since the early 1980s. *See* Lucas, 2002, Lepr Rev: 73(1):64-71. HIV/AIDS is now the leading cause of death in sub-Saharan Africa, and is the fourth biggest killer worldwide. At the end of 2001, an estimated 40 million people were living with HIV globally. *See* Norris, 2002, Radiol Technol. 73(4):339-363.

Modem anti-HIV drugs target different stages of the HIV life cycle and a variety of enzymes essential for HIV's replication and/or survival. Amongst the drugs that have so far been approved for AIDS therapy are nucleoside reverse transcriptase inhibitors ("NRTIs") such as AZT, ddI, ddC, d4T, 3TC, and abacavir; nucleotide reverse transcriptase inhibitors such as tenofovir; non-nucleoside reverse transcriptase inhibitors ("NNRTIs") such as nevirapine, efavirenz, and delavirdine; protease inhibitors ("PIs") such as saquinavir, ritonavir, indinavir, nelfinavir, amprenavir, lopinavir and atazanavir; and fusion inhibitors, such as enfuvirtide.

Nonetheless, in the vast majority of subjects none of these antiviral drugs, either alone or in combination, proves effective either to prevent eventual progression of chronic HIV infection to AIDS or to treat acute AIDS. This phenomenon is due, in part, to the high mutation rate of HIV and the rapid emergence of mutant HIV strains that are resistant to antiviral therapeutics upon administration of such drugs to infected individuals.

Many such mutant strains have been characterized in order to correlate presence of the mutations in the strains with resistant or susceptible phenotypes. For example, the V82A mutation in protease is known to correlate with resistance to a number of PIs, including, for example, SQV, RTV, and IDV. *See, e.g.,* De Clercq, 1997, Int. J. Antimicrob. Agents 353:2195-9. Further, resistance to PIs such as nelfinavir has been shown to be clinically relevant to treatment of patients infected with HIV-1 resistant to treatment with such agents. *See,* for example, Patick et al., 1998, Antimicrob. Ag. and Chemother. 42: 2637-2644.

Though numerous HIV mutations associated with both resistance and susceptibility to particular anti-viral agents have been identified, the complete set of mutations associated with PI resistance has not been completely elucidated. Indeed, the majority of clinical samples that demonstrate reduced PI susceptibility have one or more "primary" PI-selected resistance-associated mutations (RAMs). However, occasionally samples are observed with heretofore unexplained PI resistance. Identification of additional mutations associated with either resistance or susceptibility to PIs is therefore needed in order to better understand the genotypic basis of PI resistance and to guide selection of particular antiviral agents in making therapeutic decisions in the treatment of HIV-infected individuals. Further, in view of the clinical relevance of PI resistance, a more complete understanding of mutations associated with such resistance is also needed. For the first time, these, as well as other unmet needs, will be achievable as a result of the invention described hereinafter.

### 3. SUMMARY OF THE INVENTION

The present invention provides methods and compositions for determining whether an HIV-1 is resistant to a PI. In the methods resistance to a PI can be determined by detecting the presence of mutations that correlate with resistance to a PI.

Thus, in certain aspects, the invention provides a method for determining whether an HIV-1 is resistant to a PI, comprising detecting whether a mutation in at least one of codon 22, 69, 74, or 83 is present in a gene encoding protease of the HIV-1, wherein the presence of the mutation correlates with resistance to a PI, thereby determining whether the HIV-1 is resistant to the PI. In certain embodiments, the methods comprise detecting whether a mutation in at least one of codon 22, 69, 74, or 83 is present in a gene encoding protease of the HIV-1 in combination with a mutation in at least one of codon 10, 13, 19, 20, 36, 37, 54, 71, 73, 82, 88, or 93, wherein the presence of the mutations correlates with resistance to a PI, thereby determining whether the HIV-1 is resistant to the PI. The presence of the mutations associated with resistance to a PI can be detected according to any method known to one of skill in the art without limitation. Methods for detecting such mutations are described extensively below.

In other aspects, the invention provides a method for determining whether an HIV-1 is resistant to a PI, comprising detecting whether a mutation in at least one of codon 22, 69; 74, or 83 is present in a gene encoding protease of the HIV-1 in combination with a mutation in at least one of codon 23, 24, 30, 32, 46, 47, 48, 50, 54, 82, 84, 88, or 90, wherein the presence of the mutations correlates with resistance to a PI, thereby determining whether the HIV-1 is resistant to the PI. The presence of the mutations associated with resistance to a PI can be detected according to any method known to one of skill in the art without limitation. Methods for detecting such mutations are described extensively below.

In still other aspects, the invention provides a method for determining whether an HIV-1 is resistant to a PI, comprising detecting whether a mutation in at least one of codon 418 or 482 is present in a gene encoding gag of the HIV-1, wherein the presence of the mutation correlates with resistance to a PI, thereby determining whether the HIV-1 is resistant to the PI. In certain embodiments, the methods comprise detecting whether a mutation in at least one of codon 418 or 482 is present in a gene encoding gag of the HIV-1 in combination with a mutation in at least one of codon 431, 437, 449, or 453, wherein the presence of the mutations correlates with resistance to a PI, thereby determining whether the HIV-1 is resistant to the PI. The presence of the mutations associated with resistance to a PI can be detected according to any method known to one of skill in the art without limitation. Methods for detecting such mutations are described extensively below.

In yet other aspects, the invention provides a method for determining whether an HIV-1 is resistant to a PI, comprising detecting whether at least one of codon 418 or 482 is present in a gene encoding gag of the HIV-1and whether mutation in at least one of codon 22, 69, 74, or 83 is present in a gene encoding protease of the HIV-1, wherein the presence of the mutations correlates with resistance to a PI, thereby determining whether the HIV-1 is resistant to the PI. In certain embodiments, the methods further comprise detecting whether a mutation in at least one of codon 10, 13, 19, 20, 36,37, 54, 71, 73, 82, 88, or 93 is present in a gene encoding protease of the HIV-1 in combination with the gag and protease mutations, wherein the presence of the mutations correlates with resistance to a PI, thereby determining whether the HIV-1 is resistant to the PI In certain embodiments, the method further comprises detecting whether a mutation in at least one of codon 23, 24, 30, 32, 46, 47, 48, 50, 54, 82, 84, 88, or 90 is present in a gene encoding protease of the HIV-1 in combination with the gag and protease mutations, wherein the presence of the mutations correlates with resistance to a PI, thereby determining whether the HIV-1 is resistant to the PI. In certain embodiments, the methods further comprise detecting whether a mutation in at least one of codon 431, 437, 449, or 453 is present in a gene encoding gag of the HIV-1 in combination with the gag and protease mutations, wherein the presence of the mutations correlates with resistance to a PI, thereby determining whether the HIV-1 is resistant to the PI. The presence of the mutations associated with resistance to a PI can be detected according to any method known to one of skill in the art without limitation. Methods for detecting such mutations are described extensively below.

### 4. BRIEF DESCRIPTION OF THE FIGURES

Figure 1 presents a scatter diagram presenting the fold change to IC₅₀ observed for 125 clinical viral isolates relative to reference strain NL4-3 that do not comprise any primary PI resistance-associated mutations.

Figure 2 presents an exemplary classification and regression tree that can be used to identify mutations associated with PI resistance.

Figure 3 presents a phenotypic analysis of an exemplary patient sample showing phenotypic resistance to all tested PIs as well as the genotype of the sample HIV's protease mutations.

Figure 4 presents the results of phenotypic analysis of individual clones isolated from patient sample 848 to assess the relative contributions of the L19I and V82I mutations to PI resistance.

Figure 5 presents scatter diagrams showing the distribution in susceptibility to PIs of two groups of samples defined based on the presence (Figure 5A) or absence (Figure 5B) of primary PI mutations I54V, V82A, F, S, or T, and L90M, with or without M46I or L.

Figures 6A-B present a table showing gag mutations significantly correlated with altered susceptibility (e.g., resistance) to protease inhibitors.

Figures 7A-H present table showing protease mutations significantly correlated with altered susceptibility (e.g., resistance) to protease inhibitors.

Figure 8 presents a table showing mutations identified as significantly correlating with altered susceptibility (*e.g.,* resistance) to protease inhibitors by both of two different methods (described in detail in Examples 2 and 4).

### 5. DETAILED DESCRIPTION OF THE INVENTION

The present invention provides methods and compositions for determining whether an HIV-1 is resistant to antiviral therapy with a PI. The methods generally comprise detecting the presence of mutations in the HIV-1 gene encoding protease or gag that significantly correlate with resistance to a PI.

### 5.1. Abbreviations

"NRTI" is an abbreviation for nucleoside reverse transcriptase inhibitor.

"NNRTI" is an abbreviation for non nucleoside reverse transcriptase inhibitor.

"PI" is an abbreviation for protease inhibitor.

"PR" is an abbreviation for protease.

"RT" is an abbreviation for reverse transcriptase.

"PCR" is an abbreviation for "polymerase chain reaction."

"HBV" is an abbreviation for hepatitis B virus.

"HCV" is an abbreviation for hepatitis C virus.

"HIV" is an abbreviation for human immunodeficiency virus.

"AMP" is an abbreviation for the PI amprenavir.

"IDV" is an abbreviation for the PI indinavir.

"NFV" is an abbreviation for the PI nelfinavir.

"RTV" is an abbreviation for the PI ritonavir.

"SQV" is an abbreviation for the PI saquinavir.

"LPV" is an abbreviation for the PI lopinavir.

"ATV" is an abbreviation for the PI atazanavir.

The amino acid notations used herein for the twenty genetically encoded L-amino acids are conventional and are as follows:

| **Amino Acid** | **One-Letter Abbreviation** | **Three Letter Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

Unless noted otherwise, when polypeptide sequences are presented as a series of one-letter and/or three-letter abbreviations, the sequences are presented in the N → C direction, in accordance with common practice.

Individual amino acids in a sequence are represented herein as AN, wherein A is the standard one letter symbol for the amino acid in the sequence, and N is the position in the sequence. Mutations are represented herein as A₁NA₂, wherein A₁ is the standard one letter symbol for the amino acid in the reference protein sequence, A₂ is the standard one letter symbol for the amino acid in the mutated protein sequence, and N is the position in the amino acid sequence. For example, a G25M mutation represents a change from glycine to methionine at amino acid position 25. Mutations may also be represented herein as NA₂, wherein N is the position in the amino acid sequence and A₂ is the standard one letter symbol for the amino acid in the mutated protein sequence (*e.g.,* 25M, for a change from the wild-type amino acid to methionine at amino acid position 25). Additionally, mutations may also be represented herein as A₁NX, wherein A₁ is the standard one letter symbol for the amino acid in the reference protein sequence, N is the position in the amino acid sequence, and X indicates that the mutated amino acid can be any amino acid (e.g., G25X represents a change from glycine to any amino acid at amino acid position 25). This notation is typically used when the amino acid in the mutated protein sequence is either not known or, if the amino acid in the mutated protein sequence could be any amino acid, except that found in the reference protein sequence. The amino acid positions are numbered based on the full-length sequence of the protein from which the region encompassing the mutation is derived. Representations of nucleotides and point mutations in DNA sequences are analogous.

The abbreviations used throughout the specification to refer to nucleic acids comprising specific nucleobase sequences are the conventional one-letter abbreviations. Thus, when included in a nucleic acid, the naturally occurring encoding nucleobases are abbreviated as follows: adenine (A), guanine (G), cytosine (C), thymine (T) and uracil (U). Unless specified otherwise, single-stranded nucleic acid sequences that are represented as a series of one-letter abbreviations, and the top strand of double-stranded sequences, are presented in the 5' → 3' direction.

### 5.2. Definitions

As used herein, the following terms shall have the following meanings:

A "phenotypic assay" is a test that measures a phenotype of a particular virus, such as, for example, HIV, or a population of viruses, such as, for example, the population of HIV infecting a subject. The phenotypes that can be measured include, but are not limited to, the resistance or susceptibility of a virus, or of a population of viruses, to a specific anti-viral agent or that measures the replication capacity of a virus.

A "genotypic assay" is an assay that determines a genotype of an organism, a part of an organism, a population of organisms, a gene, a part of a gene, or a population of genes. Typically, a genotypic assay involves determination of the nucleic acid sequence of the relevant gene or genes. Such assays are frequently performed in HIV to establish, for example, whether certain mutations are associated with drug resistance or resistance or altered replication capacity are present.

As used herein, "genotypic data" are data about the genotype of, for example, a virus. Examples of genotypic data include, but are not limited to, the nucleotide or amino acid sequence of a virus, a population of viruses, a part of a virus, a viral gene, a part of a viral gene, or the identity of one or more nucleotides or amino acid residues in a viral nucleic acid or protein.

The term "% sequence identity" is used interchangeably herein with the term "% identity" and refers to the level of amino acid sequence identity between two or more peptide sequences or the level of nucleotide sequence identity between two or more nucleotide sequences, when aligned using a sequence alignment program. For example, as used herein, 80% identity means the same thing as 80% sequence identity determined by a defined algorithm, and means that a given sequence is at least 80% identical to another length of another sequence. Exemplary levels of sequence identity include, but are not limited to, 60, 70, 80, 85, 90, 95, 98% or more sequence identity to a given sequence.

The term "% sequence homology" is used interchangeably herein with the term "% homology" and refers to the level of amino acid sequence homology between two or more peptide sequences or the level of nucleotide sequence homology between two or more nucleotide sequences, when aligned using a sequence alignment program. For example, as used herein, 80% homology means the same thing as 80% sequence homology determined by a defined algorithm, and accordingly a homologue of a given sequence has greater than 80% sequence homology over a length of the given sequence. Exemplary levels of sequence homology include, but are not limited to, 60, 70, 80, 85, 90, 95, 98% or more sequence homology to a given sequence.

Exemplary computer programs which can be used to determine identity between two sequences include, but are not limited to, the suite of BLAST programs, e.g., BLASTN, BLASTX, and TBLASTX, BLASTP and TBLASTN, publicly available on the Internet at the NCBI website. *See also* Altschul et al., 1990, J. Mol. Biol. 215:403-10 (with special reference to the published default setting, i.e., parameters w=4, t=17) and Altschul et al., 1997, Nucleic Acids Res., 25:3389-3402. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. *See id.*

A preferred alignment of selected sequences in order to determine "% identity" between two or more sequences, is performed using for example, the CLUSTAL-X program, operated with default parameters, including an open gap penalty of 10.0, an extended gap penalty of 0.1, and a BLOSUM 30 similarity matrix.

"Polar Amino Acid" refers to a hydrophilic amino acid having a side chain that is uncharged at physiological pH, but which has at least one bond in which the pair of electrons shared in common by two atoms is held more closely by one of the atoms. Genetically encoded polar amino acids include Asn (N), Gln (Q) Ser (S) and Thr (T).

"Nonpolar Amino Acid" refers to a hydrophobic amino acid having a side chain that is uncharged at physiological pH and which has bonds in which the pair of electrons shared in common by two atoms is generally held equally by each of the two atoms (i.e., the side chain is not polar). Genetically encoded nonpolar amino acids include Ala (A), Gly (G), Ile (I), Leu (L), Met (M) and Val (V).

"Hydrophilic Amino Acid" refers to an amino acid exhibiting a hydrophobicity of less than zero according to the normalized consensus hydrophobicity scale of Eisenberg et al., 1984, J. Mol. Biol. 179:125-142. Genetically encoded hydrophilic amino acids include Arg (R), Asn (N), Asp (D), Glu (E), Gln (Q), His (H), Lys (K), Ser (S) and Thr (T).

"Hydrophobic Amino Acid" refers to an amino acid exhibiting a hydrophobicity of greater than zero according to the normalized consensus hydrophobicity scale of Eisenberg et al., 1984, J. Mol. Biol. 179;125-142, Genetically encoded hydrophobic amino acids include Ala (A), Gly (G), Ile (I), Leu (L), Met (M), Phe (F), Pro (P), Trp (W), Tyr (Y) and Val (V).

"Acidic Amino Acid" refers to a hydrophilic amino acid having a side chain pK value of less than 7. Acidic amino acids typically have negatively charged side chains at physiological pH due to loss of a hydrogen ion. Genetically encoded acidic amino acids include Asp (D) and Glu (E).

"Basic Amino Acid" refers to a hydrophilic amino acid having a side chain pK value of greater than 7. Basic amino acids typically have positively charged side chains at physiological pH due to association with a hydrogen ion. Genetically encoded basic amino acids include Arg (R), His (H) and Lys (K).

A "mutation" is a change in an amino acid sequence or in a corresponding nucleic acid sequence relative to a reference nucleic acid or polypeptide. For embodiments of the invention comprising HIV protease or reverse transcriptase, the reference nucleic acid encoding protease or reverse transcriptase is the protease or reverse transcriptase coding sequence, respectively, present in NL4-3 HIV (GenBank Accession No. AF324493). Likewise, the reference protease or reverse transcriptase polypeptide is that encoded by the NL4-3 HIV sequence. Although the amino acid sequence of a peptide can be determined directly by, for example, Edman degradation or mass spectroscopy, more typically, the amino sequence of a peptide is inferred from the nucleotide sequence of a nucleic acid that encodes the peptide. Any method for determining the sequence of a nucleic acid known in the art can be used, for example, Maxam-Gilbert sequencing (Maxam et al., 1980, Methods in Enzymology 65:499), dideoxy sequencing (Sanger et al., 1977, Proc. Natl. Acad. Sci. USA 74:5463) or hybridization-based approaches (*see e.g.,* Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY).

A "mutant" is a virus, gene or protein having a sequence that has one or more changes relative to a reference virus, gene or protein.

The terms "peptide," "polypeptide" and "protein" are used interchangeably throughout.

The term "wild-type" refers to a viral genotype that does not comprise a mutation known to be associated with drug resistance.

The terms "polynucleotide," "oligonucleotide" and "nucleic acid" are used interchangeably throughout.

### 5.3. Methods of Determining Resistance to a PI

In certain aspects, the present invention provides methods for determining whether an HIV-1 is resistant or susceptible to a PI. In general, the methods comprise detecting whether a mutation or combination of mutations significantly correlated with PI resistance are present in the gene encoding protease or gag of the HIV-1, as demonstrated by the examples below.

Therefore, in certain embodiments, the invention provides a method for determining whether an HIV-1 is resistant to a PI, comprising detecting whether a mutation in at least one of codon 22, 69, 74, or 83 is present in a gene encoding protease of the HIV-1, wherein the presence of the mutation correlates with resistance to a PI, thereby determining whether the HIV-1 is resistant to the PI In certain embodiments, the methods comprise detecting whether a mutation in at least two, three, or four of codon 22, 69, 74, or 83 is present in a gene encoding protease of the HIV-1. In certain embodiments, the methods comprise detecting whether a mutation at codon 22 is present in the gene encoding protease of the HIV-1. In certain embodiments, the methods comprise detecting whether a mutation at codon 69 is present in the gene encoding protease of the HIV-1. In certain embodiments, the methods comprise detecting whether a mutation at codon 74 is present in the gene encoding protease of the HIV-1. In certain embodiments, the methods comprise detecting whether a mutation at codon 83 is present in the gene encoding protease of the HIV-1. In certain embodiments, the mutation at codon 22 encodes valine (V). In certain embodiments, the mutation at codon 69 encodes arginine (R). In certain embodiments, the mutation at codon 74 encodes lysine (K) or serine (S). In certain embodiments, the mutation at codon 83 encodes aspartic acid (D).

In certain embodiments, the method further comprises detecting whether a mutation in at least one of codon 10, 13, 19, 20, 36, 37, 54, 71, 73, 82, 88, or 93 is present in a gene encoding protease of the HIV-1 in combination with the mutation in at least one of codon 22, 69, 74, or 83 in the gene encoding HIV-1 protease, wherein the presence of the mutations correlates with resistance to a PI, thereby determining whether the HIV-1 is resistant to the PI. In certain embodiments, the methods comprise detecting whether a mutation in at least two, three, four, five, six, seven, eight, nine, ten, eleven or twelve of codon 10, 13, 19, 20, 36, 37, 54, 71, 73, 82, 88, or 93 is present in a gene encoding protease of the HIV-1. In certain embodiments, the methods comprise detecting whether a mutation at codon 10 is present. In certain embodiments, the methods comprise detecting whether a mutation at codon 13 is present. In certain embodiments, the methods comprise detecting whether a mutation at codon 19 is present. In certain embodiments, the methods comprise detecting whether a mutation at codon 20 is present. In certain embodiments, the methods comprise detecting whether a mutation at codon 36 is present. In certain embodiments, the methods comprise detecting whether a mutation at codon 37 is present. In certain embodiments, the methods comprise detecting whether a mutation at codon 54 is present. In certain embodiments, the methods comprise detecting whether a mutation at codon 71 is present. In certain embodiments, the methods comprise detecting whether a mutation at codon 73 is present. In certain embodiments, the methods comprise detecting whether a mutation at codon 82 is present. In certain embodiments, the methods comprise detecting whether a mutation at codon 88 is present. In certain embodiments, the methods comprise detecting whether a mutation at codon 93 is present. In certain embodiments, the mutation at codon 10 encodes isoleucine (I) or valine (V). In certain embodiments, the mutation at codon 13 encodes valine (V). In certain embodiments, the mutation at codon 19 encodes isoleucine (I) or valine (V). In certain embodiments, the mutation at codon 20 encodes isoleucine (I), methionine (M), or threonine (T). In certain embodiments, the mutation at codon 36 encodes isoleucine (I) or valine (V). In certain embodiments, the mutation at codon 37 encodes aspartic acid (D). In certain embodiments, the mutation at codon 54 encodes valine (V). In certain embodiments, the mutation at codon 71 encodes isoleucine (I), threonine (T), or valine (V). In certain embodiments, the mutation at codon 73 encodes serine (S). In certain embodiments, the mutation at codon 82 encodes isoleucine (I). In certain embodiments, the mutation at codon 88 encodes aspartic acid (D). In certain embodiments, the mutation at codon 93 encodes leucine (L).

In certain embodiments, the method further comprises detecting whether a mutation in at least one of codon 23, 24, 30, 32, 46, 47, 48, 50, 54, 82, 84, 88, or 90 is present in a gene encoding protease of the HIV-1 in combination with the mutation in at least one of codon 22, 69, 74, or 83 in the HIV-1 protease gene, wherein the presence of the mutations correlates with resistance to a PI, thereby determining whether the HIV-1 is resistant to the PI. In certain embodiments, the methods comprise detecting whether a mutation at codon 23 is present. In certain embodiments, the methods comprise detecting whether a mutation in at least two, three, four, five, six, seven, eight, nine, ten, eleven, twelve, or thirteen of codon 23, 24, 30, 32, 46, 47, 48, 50, 54, 82, 84, 88, or 90 is present in a gene encoding protease of the HIV-1. In certain embodiments, the methods comprise detecting whether a mutation at codon 24 is present. In certain embodiments, the methods comprise detecting whether a mutation at codon 30 is present. In certain embodiments, the methods comprise detecting whether a mutation at codon 32 is present. In certain embodiments, the methods comprise detecting whether a mutation at codon 46 is present. In certain embodiments, the methods comprise detecting whether a mutation at codon 47 is present. In certain embodiments, the methods comprise detecting whether a mutation at codon 48 is present. In certain embodiments, the methods comprise detecting whether a mutation at codon 50 is present. In certain embodiments, the methods comprise detecting whether a mutation at codon 54 is present. In certain embodiments, the methods comprise detecting whether a mutation at codon 82 is present. In certain embodiments, the methods comprise detecting whether a mutation at codon 84 is present. In certain embodiments, the methods comprise detecting whether a mutation at codon 88 is present. In certain embodiments, the methods comprise detecting whether a mutation at codon 90 is present. In certain embodiments, the mutation at codon 23 encodes isoleucine (I). In certain embodiments, the mutation at codon 24 encodes isoleucine (I) or valine (V). In certain embodiments, the mutation at codon 30 encodes asparagine (N). In certain embodiments, the mutation at codon 32 encodes alanine (A) or isoleucine (I). In certain embodiments, the mutation at codon 46 encodes phenylalanine (F), isoleucine (I), leucine (L), or valine (V). In certain embodiments, the mutation at codon 47 encodes alanine (A) or valine (V). In certain embodiments, the mutation at codon 48 encodes alanine (A), glutamic acid (E), leucine (L), methionine (M), or valine (V). In certain embodiments, the mutation at codon 50 encodes leucine (L) or valine (V). In certain embodiments, the mutation at codon 54 encodes alanine (A), leucine (L), serine (S), threonine (T), methionine (M), or valine (V). In certain embodiments, the mutation at codon 82 encodes threonine (T), alanine (A), phenylalanine (F), or serine (S). In certain embodiments, the mutation at codon 84 encodes cysteine (C), alanine (A), or valine (V). In certain embodiments, the mutation at codon 88 encodes serine (S) or threonine (T). In certain embodiments, the mutation at codon 90 encodes methionine (M).

In another aspect, the invention provides a method for determining whether an HIV-1 is resistant to a PI, comprising detecting whether a mutation in at least one of codon 418 or 482 is present in a gene encoding gag of the HIV-1, wherein the presence of the mutation correlates with resistance to a PI, thereby determining whether the HIV-1 is resistant to the PI. In certain embodiments, the methods comprise detecting whether a mutation at codon 418 is present. In certain embodiments, the methods comprise detecting whether a mutation at codon 482 is present. In certain embodiments, the methods comprise detecting whether mutations at codons 418 and 482 are present. In certain embodiments, the mutation at codon 418 encodes glutamic acid (E) or arginine (R). In certain embodiments, the mutation at codon 482 encodes glycine (G).

In certain embodiments, the method further comprises detecting whether a mutation in at least one of codon 431, 437, 449, or 453 is present in a gene encoding gag of the HIV-1 in combination with the mutation in at least one of codon 418 or 482 of HIV-1 gag, wherein the presence of the mutations correlates with resistance to a PI, thereby determining whether the HIV-1 is resistant to the PI. In certain embodiments, the methods comprise detecting whether a mutation in at least two, three, or four, of codon 431, 437, 449, or 453 is present in a gene encoding gag of the HIV-1. In certain embodiments, the methods comprise detecting whether a mutation at codon 431 is present. In certain embodiments, the methods comprise detecting whether a mutation at codon 437 is present. In certain embodiments, the methods comprise detecting whether a mutation at codon 449 is present. In certain embodiments, the methods comprise detecting whether a mutation at codon 453 is present. In certain embodiments, the mutation at codon 431 encodes valine (V). In certain embodiments, the mutation at codon 437 encodes valine (V). In certain embodiments, the mutation at codon 449 encodes isoleucine (I) or proline (P). In certain embodiments, the mutation at codon 453 encodes leucine (L).

In yet other aspects, the invention provides a method for determining whether an HIV-1 is resistant to a PI, comprising detecting whether at least one of codon 418 or 482 is present in a gene encoding gag of the HIV-1and/or whether mutation in at least one of codon 22, 69, 74, or 83 is present in a gene encoding protease of the HIV-1, wherein the presence of the mutations correlates with resistance to a PI, thereby determining whether the HIV-1 is resistant to the PI. In certain embodiments, the methods further comprise detecting whether a mutation in at least one of codon 10,13,19,20, 36, 37, 54,71,73, 82, 88, or 93 is present in a gene encoding protease of the HIV-1 in combination with the gag and protease mutations, wherein the presence of the mutations correlates with resistance to a PI, thereby determining whether the HIV-1 is resistant to the PI In certain embodiments, the method further comprises detecting whether a mutation in at least one of codon 23, 24, 30, 32, 46, 47, 48, 50, 54, 82, 84, 88, or 90 is present in a gene encoding protease of the HIV-1 in combination with the gag and protease mutations, wherein the presence of the mutations correlates with resistance to a PI, thereby determining whether the HIV-1 is resistant to the PI. In certain embodiments, the methods further comprise detecting whether a mutation in at least one of codon 431, 437, 449, or 453 is present in a gene encoding gag of the HIV-1 in combination with the gag and protease mutations, wherein the presence of the mutations correlates with resistance to a PI, thereby determining whether the HIV-1 is resistant to the PI. The presence of the mutations associated with resistance to a PI can be detected according to any method known to one of skill in the art without limitation. Methods for detecting such mutations are described extensively below.

In certain embodiments, the PI is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

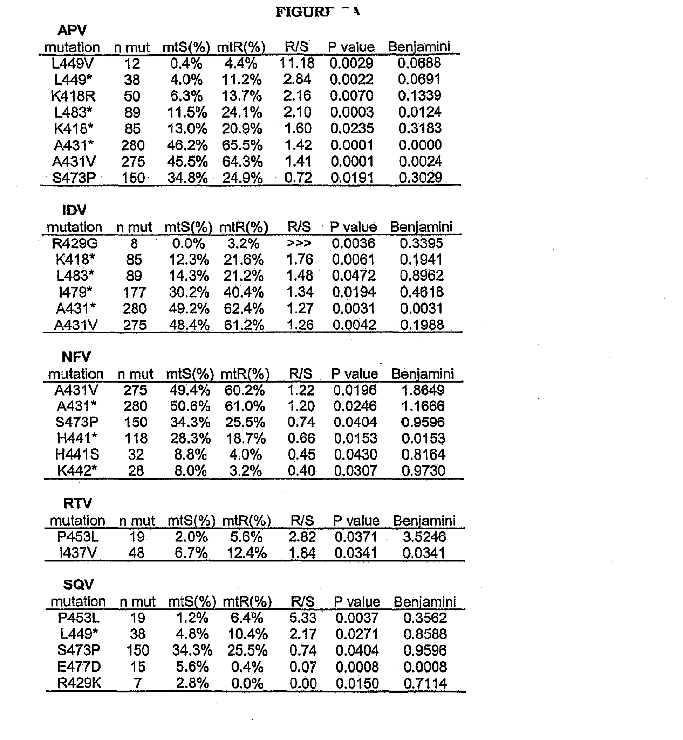

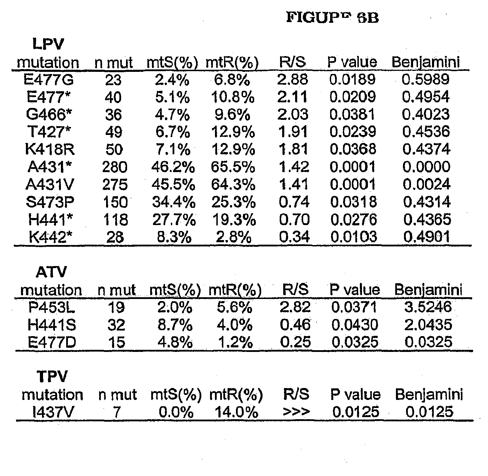

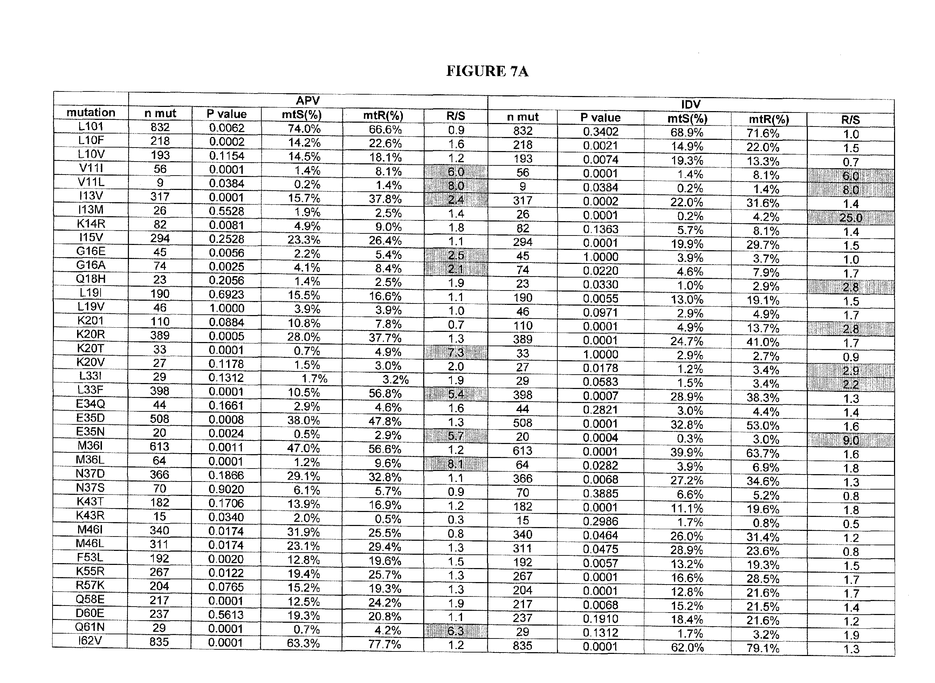

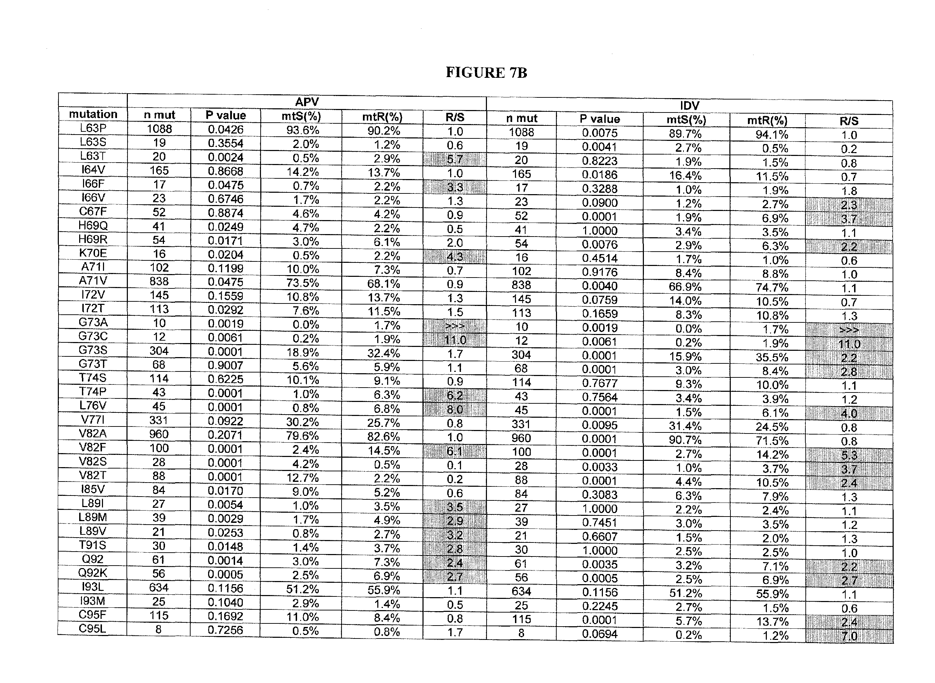

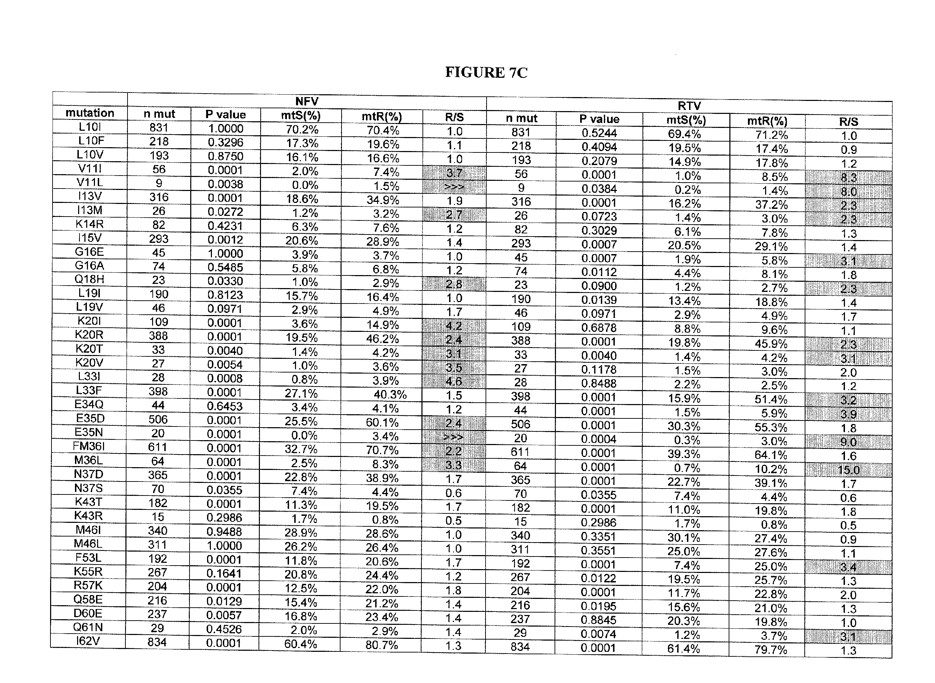

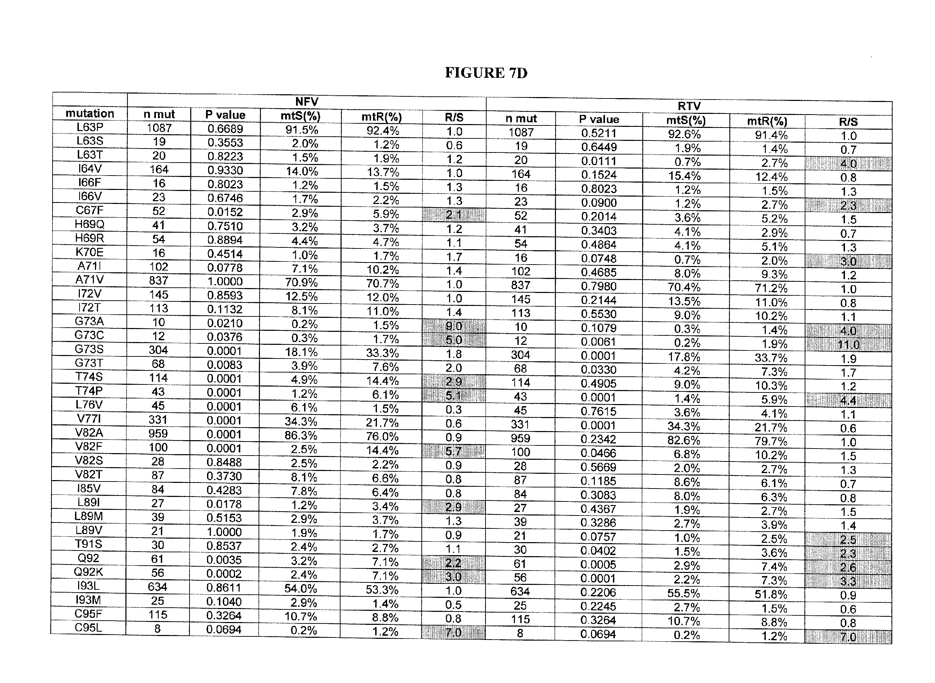

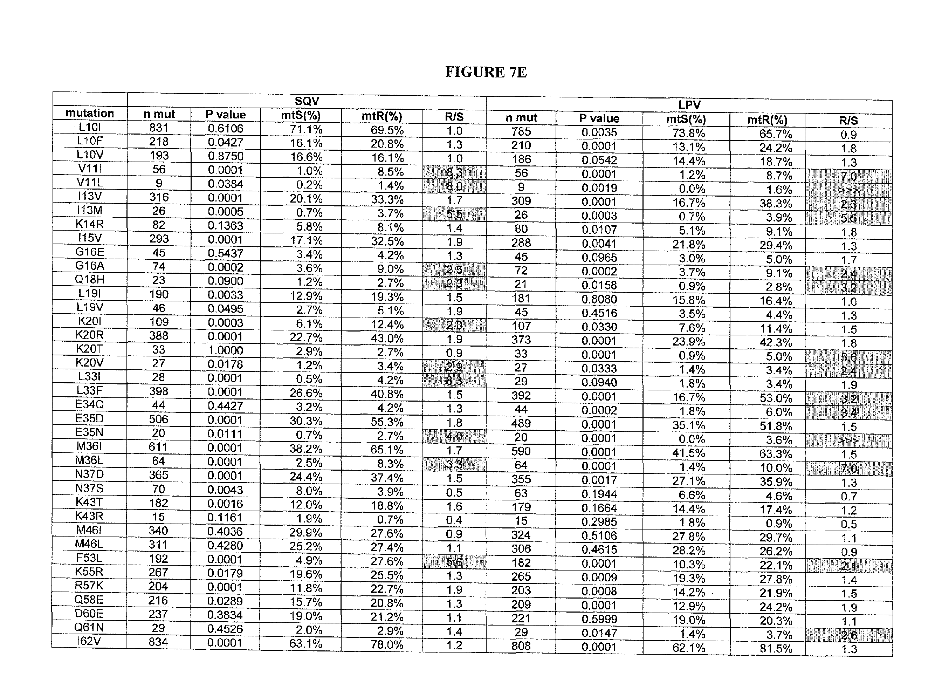

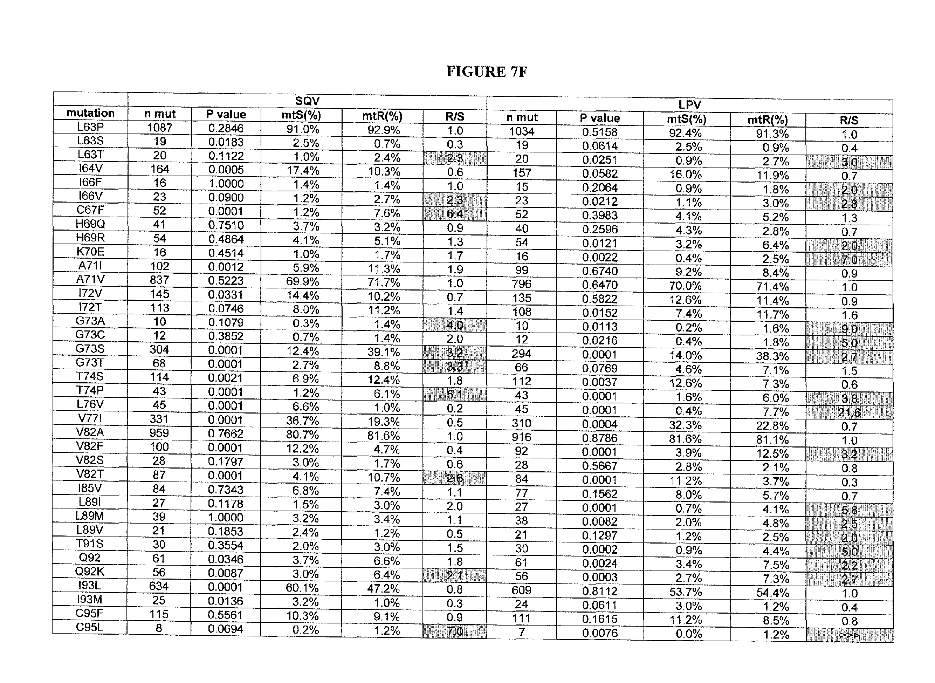

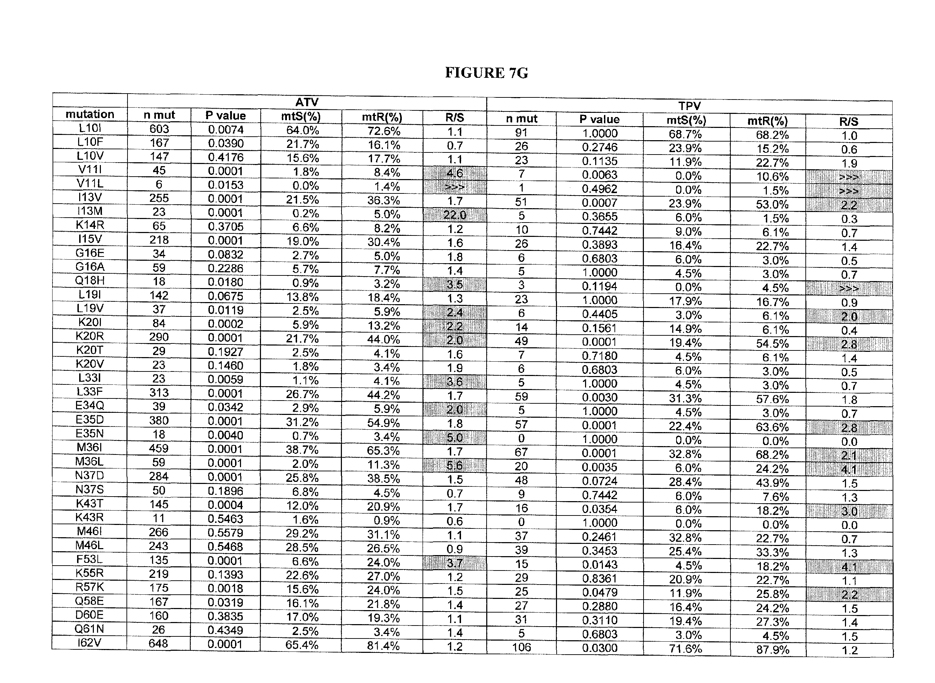

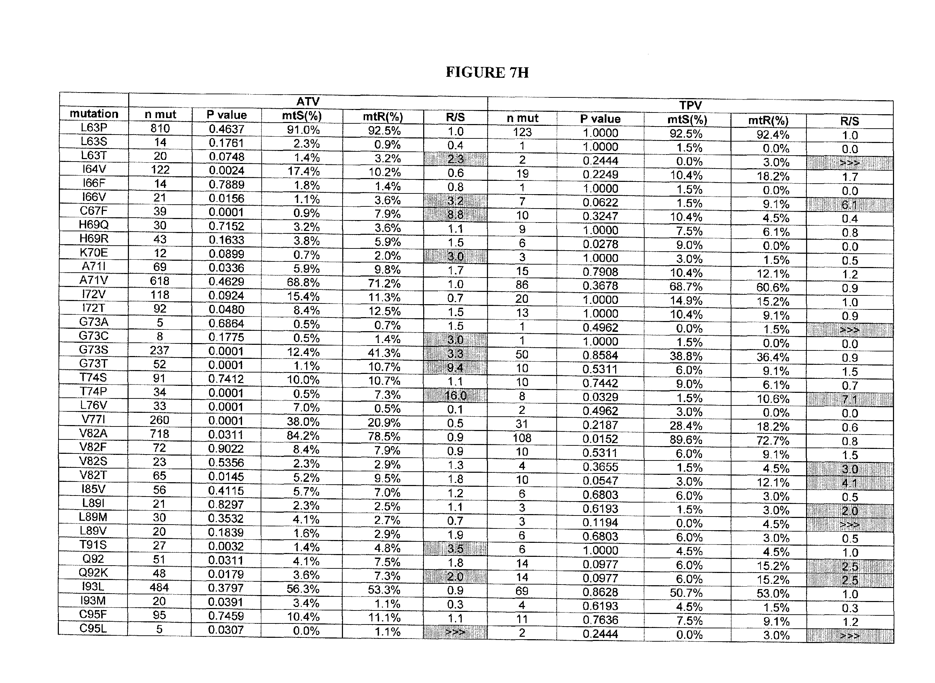

In yet other embodiments, the method for determining whether an HIV-1 is resistant to a PI comprises detecting whether a mutation or a combination of mutations significantly associated with PI resistance as indicated by Table 3 or 4 or Figures 6 or 7 is present in a gene encoding protease or gag of the HIV-1. In certain embodiments, the mutation is in a codon that, when mutated, is significantly associated with PI resistance. For example, Table 4 indicates that the gag mutation K418E is significantly associated with PI resistance. In certain embodiments, therefore, the methods comprise determining whether any mutation in gag codon 418 is present.

In certain embodiments, the significance of the association of the mutation or combination of mutations with PI resistance is indicated by an odds ratio greater than 1. In certain embodiments, the significance of the association of the mutation or combination of mutations with PI resistance is indicated by a p-value calculated with Fisher's Exact Test less than 0.05. In a preferred embodiment, the significance of the association of the mutation or combination of mutations with PI resistance is indicated by a p-value calculated with Fisher's Exact Test less than 0.01, still more preferably, less than 0.005. In certain embodiments, the HIV-1 is resistant to IDV, NFV, RTV, SQV, LPV, or ATV. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In another aspect, the methods comprise determining whether a subject is infected with an HIV that is resistant to a PI according to a method of the invention, then advising a medical professional of the treatment option of administering to the subject a therapeutic regimen that does not include the PI. In certain embodiments, the PI is AMP, IDV, NFV, RTV, SQV, LPV, or ATV.

In another aspect, the methods comprise determining whether a subject is infected with an HIV that is resistant to a PI according to a method of the invention, then advising a medical professional to treat the subject with a therapeutic regimen that does not include the PI. In certain embodiments, the PI is AMP, IDV, NFV, RTV, SQV, LPV, or ATV.

In still another aspect, the methods comprise determining whether a subject is infected with an HIV that is resistant to a PI according to a method of the invention, and administering to the subject a combination of anti-HIV agents that does not include the PI. In certain embodiments, the PI is AMP, IDV, NFV, RTV, SQV, LPV, or ATV.

In still another aspect, the methods comprise determining whether a subject is infected with an HIV that is resistant to a PI according to a method of the invention, then advising a medical professional of the treatment option of administering to the subject a combination of anti-HIV agents that does not include the PI. In certain embodiments, the PI is AMP, IDV, NFV, RTV, SQV, LPV, or ATV.

In another aspect, the methods comprise determining whether a subject is infected with an HIV that is resistant to a PI according to a method of the invention, then advising a medical professional of the treatment option of administering to the subject a therapeutic regimen that comprises the PI. In certain embodiments, the PI is AMP, IDV, NFV, RTV, SQV, LPV, or ATV. In such embodiments, the mutation associated with resistance to the PI preferably also impairs the replication capacity of the HIV-1. Such mutations are extensively described in U.S. Patent Application No. 11/052,741, filed February 4, 2005, which is hereby incorporated by reference in its entirety.

In another aspect, the methods comprise determining whether a subject is infected with an HIV that is resistant to a PI according to a method of the invention, then advising a medical professional to treat the subj ect with a therapeutic regimen that comprises the PI. In certain embodiments, the PI is AMP, IDV, NFV, RTV, SQV, LPV, or ATV. In such embodiments, the mutation associated with resistance to the PI preferably also impairs the replication capacity of the HIV-1.

In still another aspect, the methods comprise determining whether a subject is infected with an HIV that is resistant to a PI according to a method of the invention, and administering to the subject a combination of anti-HIV agents comprises the PI. In certain embodiments, the PI is AMP, IDV, NFV, RTV, SQV, LPV, or ATV. In such embodiments, the mutation associated with resistance to the PI preferably also impairs the replication capacity of the HIV-1.

In still another aspect, the methods comprise determining whether a subject is infected with an HIV that is resistant to a PI according to a method of the invention, then advising a medical professional of the treatment option of administering to the subject a combination of anti-HIV agents that comprises the PI. In certain embodiments, the PI is AMP, IDV, NFV, RTV, SQV, LPV, or ATV. In such embodiments, the mutation associated with resistance to the PI preferably also impairs the replication capacity of the HIV-1.

In still another aspect, the methods comprise determining whether a subject is infected with an HIV that is resistant to a PI according to a method of the invention at a first time, then determining whether the subject remains infected with an HIV that is resistant to a PI according to a method of the invention at a later second time. In other embodiments, the methods comprise determining whether a subject is infected with an HIV that is resistant to a PI according to a method of the invention at a first time, then determining whether the subject is infected with an HIV that is no longer resistant to the PI according to a method of the invention at a later second time. In still other embodiments, the methods comprise determining whether a subject is infected with an HIV that is not resistant to a PI according to a method of the invention at a first time, then determining whether the subject is infected with an HIV that is resistant to the PI according to a method of the invention at a later second time. In yet other embodiments, the methods comprise determining whether a subject is infected with an HIV that is not resistant to a PI according to a method of the invention at a first time, then determining whether the subject remains infected with an HIV that is not resistant to a PI according to a method of the invention at a later second time.

### 5.4. Measuring Resistance of HIV-1 to a PI

Any method known in the art can be used to determine a viral drug resistance phenotype, without limitation. *See e.g.,* U.S. Patent Nos. 5,837,464 and 6,242,187, each of which is hereby incorporated by reference in its entirety.

In certain embodiments, the phenotypic analysis is performed using recombinant virus assays ("RVAs"). RVAs use virus stocks generated by homologous recombination between viral vectors and viral gene sequences, amplified from the patient virus. In certain embodiments, the viral vector is a HIV vector and the viral gene sequences are protease and/or reverse transcriptase and/or gag sequences.

In preferred embodiments, the phenotypic analysis of PI resistance is performed using PHENOSENSE™ (ViroLogic Inc., South San Francisco, CA). *See* Petropoulos et al., 2000, Antimicrob. Agents Chemother. 44:920-928; U.S. Patent Nos. 5,837,464 and 6,242,187. PHENOSENSE™ is a phenotypic assay that achieves the benefits of phenotypic testing and overcomes the drawbacks of previous assays. Because the assay has been automated, PHENOSENSE™ provides high throughput methods under controlled conditions for determining PI resistance, susceptibility, or resistance of a large number of individual viral isolates.

The result is an assay that can quickly and accurately define both the replication capacity and the susceptibility profile of a patient's HIV (or other virus) isolates to all currently available antiretroviral drugs, including the PIs AMP, IDV, NFV, RTV, SQV, LPV, and ATV. PHENOSENSE™ can obtain results with only one round of viral replication, thereby avoiding selection of subpopulations of virus that can occur during preparation of viral stocks required for assays that rely on fully infectious virus. Further, the results are both quantitative, measuring varying degrees of replication capacity or antiviral resistance or susceptibility, and sensitive, as the test can be performed on blood specimens with a viral load of about 500 copies/mL or above and can detect minority populations of some drug-resistant virus at concentrations of 10% or less of total viral population. Finally, the replication capacity and antiviral drug resistance results are reproducible and can vary by less than about 0.25 logs in about 95% of the assays performed.

PHENOSENSE™ can be used with nucleic acids from amplified viral gene sequences. As discussed below, the nucleic acid can be amplified from any sample known by one of skill in the art to contain a viral gene sequence, without limitation. For example, the sample can be a sample from a human or an animal infected with the virus or a sample from a culture of viral cells. In certain embodiments, the viral sample comprises a genetically modified laboratory strain. In other embodiments, the viral sample comprises a wild-type isolate.

A resistance test vector ("RTV") can then be constructed by incorporating the amplified viral gene sequences into a replication defective viral vector by using any method known in the art of incorporating gene sequences into a vector. In one embodiment, restrictions enzymes and conventional cloning methods are used. *See* Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY. In a preferred embodiment, *Apa*I and *Pin*AI restriction enzymes are used. Preferably, the replication defective viral vector is the indicator gene viral vector ("IGVV"). In a preferred embodiment, the viral vector contains a means for detecting replication of the RTV. Preferably, the viral vector contains a luciferase expression cassette.

The assay can be performed by first co-transfecting host cells with RTV DNA and a plasmid that expresses the envelope proteins of another retrovirus, for example, amphotropic murine leukemia virus (MLV). Following transfection, viral particles can be harvested from the cell culture and used to infect fresh target cells in the presence of varying amounts of anti-viral drug(s). The completion of a single round of viral replication in the fresh target cells can be detected by the means for detecting replication contained in the vector. In a preferred embodiment, the completion of a single round of viral replication results in the production of luciferase. By monitoring the amount of, *e.g.,* luciferase activity in the presence of the varying amounts of antiviral drug(s), a resistance curve can be constructed by plotting luciferase activity versus drug concentration. The susceptibility of an HIV, or population of HIV, can be determined by measuring the concentration of antiviral drug at which the luciferase activity detected is half-maximal. This number, the IC₅₀, provides a standard and convenient measure of drug resistance.

In preferred embodiments, PHENOSENSE™ is used to evaluate the AMP, IDV, NFV, RTV, SQV, LPV, and/or ATV susceptibility phenotype of HIV-1. In other embodiments, PHENOSENSE™ is used to evaluate the AMP, IDV, NFV, RTV, SQV, LPV, and/or ATV susceptibility phenotype of HIV-2. In certain embodiments, the HIV-1 strain that is evaluated is a wild-type isolate of HIV-1. In other embodiments, the HIV-1 strain that is evaluated is a mutant strain of HIV-1. In certain embodiments, such mutant strains can be isolated from patients. In other embodiments, the mutant strains can be constructed by site-directed mutagenesis or other equivalent techniques known to one of skill in the art. In still other embodiments, the mutant strains can be isolated from cell culture. The cultures can comprise multiple passages through cell culture in the presence of antiviral compounds to select for mutations that accumulate in culture in the presence of such compounds.

In one embodiment, viral nucleic acid, for example, HIV-1 RNA is extracted from plasma samples, and a fragment of, or entire viral genes can be amplified by methods such as, but not limited to PCR. *See, e.g.,* Hertogs et al., 1998, Antimicrob Agents Chemother 42(2):269-76. In one example, a 2.2-kb fragment containing the entire HIV-1 PR- and RT-coding sequence is amplified by nested reverse transcription-PCR. The pool of amplified nucleic acid, for example, the PR-RT-coding sequences, is then cotransfected into a host cell such as CD4+ T lymphocytes (MT4) with the pGEMT3deltaPRT plasmid from which most of the PR (codons 10 to 99) and RT (codons 1 to 482) sequences are deleted. Homologous recombination leads to the generation of chimeric viruses containing viral coding sequences, such as the PR- and RT-coding sequences derived from HIV-1 RNA in plasma. The replication capacities or antiviral drug resistance phenotypes of the chimeric viruses can be determined by any cell viability assay known in the art, and compared to replication capacities or antiviral drug susceptibilities of a statistically significant number of individual viral isolates to assess whether a virus has altered replication capacity or is resistant or resistant to the antiviral drug. For example, an MT4 cell-3-(4,5-dimethylthiazol-2-yl) -2,5-diphenyltetrazolium bromide-based cell viability assay can be used in an automated system that allows high sample throughput.

In another embodiment, competition assays can be used to assess replication capacity of one viral strain relative to another viral strain. For example, two infectious viral strains can be co-cultivated together in the same culture medium. *See, e.g.,* Lu et al., 2001, JAIDS 27:7-13, which is incorporated by reference in its entirety. By monitoring the course of each viral strain's growth, the fitness of one strain relative to the other can be determined. By measuring many viruses' fitness relative to a single reference virus, an objective measure of each strain's fitness can be determined.

Other assays for evaluating the phenotypic susceptibility of a virus to anti-viral drugs known to one of skill in the art can be adapted to determine replication capacity or to determine antiviral drug susceptibility or resistance. *See, e.g.,* Shi and Mellors, 1997, Antimicrob Agents Chemother. 41(12):2781-85; Gervaix et al., 1997, Proc Natl Acad Sci U.S.A. 94(9):4653-8; Race et al., 1999, AIDS 13:2061-2068, incorporated herein by reference in their entireties.

In addition, the phenotypic assays described above can also be used to determine the effectiveness of candidate compounds. Generally, such methods comprise performing the phenotypic assay in the presence and absence of the candidate compound, wherein the difference in activity or expression of the indicator gene indicates the effectiveness of the candidate compound. Advantageously, the methods can be performed in the presence of a mutation associated with PI resistance as disclosed herein. By performing such assays in the presence of such mutations, candidate compounds can be identified that have beneficial interactions with the PIs to which the virus is resistant. In certain embodiments, the candidate compound will have an additive effect on viral inhibition with the PI. In preferred embodiments, the candidate compound will have a synergistic effect on viral inhibition with the PI. Further guidance may be found in performing the assays to determine the effectiveness of candidate compounds in Petropoulos et al., 2000, Antimicrob. Agents Chemother. 44:920-928; and U.S. Patent Nos. 5,837,464 and 6,242,187.

### 5.4.1. Detecting the Presence or Absence of Mutations in a Virus

The presence or absence of an mutation associated with PI resistance according to the present invention in a virus can be determined by any means known in the art for detecting a mutation. The mutation can be detected in the viral gene that encodes a particular protein, or in the protein itself, *i.e.,* in the amino acid sequence of the protein.

In one embodiment, the mutation is in the viral genome. Such a mutation can be in, for example, a gene encoding a viral protein, in a genetic element such as a *cis* or *trans* acting regulatory sequence of a gene encoding a viral protein, an intergenic sequence, or an intron sequence. The mutation can affect any aspect of the structure, function, replication or environment of the virus that changes its susceptibility to an anti-viral treatment and/or its replication capacity. In one embodiment, the mutation is in a gene encoding a viral protein that is the target of an currently available anti-viral treatment. In other embodiments, the mutation is in a gene or other genetic element that is not the target of a currently-available anti-viral treatment.

A mutation within a viral gene can be detected by utilizing any suitable technique known to one of skill in the art without limitation. Viral DNA or RNA can be used as the starting point for such assay techniques, and may be isolated according to standard procedures which are well known to those of skill in the art.

The detection of a mutation in specific nucleic acid sequences, such as in a particular region of a viral gene, can be accomplished by a variety of methods including, but not limited to, restriction-fragment-length-polymorphism detection based on allele-specific restriction-endonuclease cleavage (Kan and Dozy, 1978, Lancet ii:910-912), mismatch-repair detection (Faham and Cox, 1995, Genome Res 5:474-482), binding of MutS protein (Wagner et al., 1995, Nucl Acids Res 23:3944-3948), denaturing-gradient gel electrophoresis (Fisher et al., 1983, Proc. Natl. Acad. Sci. U.S.A. 80:1579-83), single-strand-conformation-polymorphism detection (Orita et al., 1983, Genomics 5:874-879), RNAase cleavage at mismatched base-pairs (Myers et al., 1985, Science 230:1242), chemical (Cotton et al., 1988, Proc. Natl. Acad. Sci. U.S.A. 85:4397-4401) or enzymatic (Youil et al., 1995, Proc. Natl. Acad. Sci. U.S.A. 92:87-91) cleavage of heteroduplex DNA, methods based on oligonucleotide-specific primer extension (Syvänen et al., 1990, Genomics 8:684-692), genetic bit analysis (Nikiforov et al., 1994, Nucl Acids Res 22:4167-4175), oligonucleotide-ligation assay (Landegren et al., 1988, Science 241:1077), oligonucleotide-specific ligation chain reaction ("LCR") (Barrany, 1991, Proc. Natl. Acad. Sci. U.S.A. 88:189-193), gap-LCR (Abravaya et al., 1995, Nucl Acids Res 23:675-682), radioactive or fluorescent DNA sequencing using standard procedures well known in the art, and peptide nucleic acid (PNA) assays (Orum et al., 1993, Nucl. Acids Res. 21:5332-5356; Thiede et al., 1996, Nucl. Acids Res. 24:983-984).

In addition, viral DNA or RNA may be used in hybridization or amplification assays to detect abnormalities involving gene structure, including point mutations, insertions, deletions and genomic rearrangements. Such assays may include, but are not limited to, Southern analyses (Southern, 1975, J. Mol. Biol. 98:503-517), single stranded conformational polymorphism analyses (SSCP) (Orita et al., 1989, Proc. Natl. Acad. Sci. USA 86:2766-2770), and PCR analyses (U.S. Patent Nos. 4,683,202; 4,683,195; 4,800,159; and 4,965,188; PCR Strategies, 1995 Innis et al. (eds.), Academic Press, Inc.).

Such diagnostic methods for the detection of a gene-specific mutation can involve for example, contacting and incubating the viral nucleic acids with one or more labeled nucleic acid reagents including recombinant DNA molecules, cloned genes or degenerate variants thereof, under conditions favorable for the specific annealing of these reagents to their complementary sequences. Preferably, the lengths of these nucleic acid reagents are at least 15 to 30 nucleotides. After incubation, all non-annealed nucleic acids are removed from the nucleic acid molecule hybrid. The presence of nucleic acids which have hybridized, if any such molecules exist, is then detected. Using such a detection scheme, the nucleic acid from the virus can be immobilized, for example, to a solid support such as a membrane, or a plastic surface such as that on a microtiter plate or polystyrene beads. In this case, after incubation, non-annealed, labeled nucleic acid reagents of the type described above are easily removed. Detection of the remaining, annealed, labeled nucleic acid reagents is accomplished using standard techniques well-known to those in the art. The gene sequences to which the nucleic acid reagents have annealed can be compared to the annealing pattern expected from a normal gene sequence in order to determine whether a gene mutation is present.

These techniques can easily be adapted to provide high-throughput methods for detecting mutations in viral genomes. For example, a gene array from Affymetrix (Affymetrix, Inc., Sunnyvale, CA) can be used to rapidly identify genotypes of a large number of individual viruses. Affymetrix gene arrays, and methods of making and using such arrays, are described in, for example, U.S. Patent Nos. 6,551,784, 6,548,257, 6,505,125, 6,489,114, 6,451,536, 6,410,229, 6,391,550, 6,379,895, 6,355,432, 6,342,355, 6,333,155, 6,308,170, 6,291,183, 6,287,850, 6,261,776, 6,225,625, 6,197,506, 6,168,948, 6,156,501, 6,141,096, 6,040,138, 6,022,963, 5,919,523, 5,837,832, 5,744,305, 5,834,758, and 5,631,734, each of which is hereby incorporated by reference in its entirety.

In addition, Ausubel et al., eds., Current Protocols in Molecular Biology, 2002, Vol. 4, Unit 25B, Ch. 22, which is hereby incorporated by reference in its entirety, provides further guidance on construction and use of a gene array for determining the genotypes of a large number of viral isolates. Finally, U.S. Patent Nos. 6,670,124; 6,617,112; 6,309,823; 6,284,465; and 5,723,320, each of which is incorporated by reference in its entirety, describe related array technologies that can readily be adapted for rapid identification of a large number of viral genotypes by one of skill in the art.

Alternative diagnostic methods for the detection of gene specific nucleic acid molecules may involve their amplification, *e.g.,* by PCR (U.S. Patent Nos. 4,683,202; 4,683,195; 4,800,159; and 4,965,188; PCR Strategies, 1995 Innis et al. (eds.), Academic Press, Inc.), followed by the detection of the amplified molecules using techniques well known to those of skill in the art. The resulting amplified sequences can be compared to those which would be expected if the nucleic acid being amplified contained only normal copies of the respective gene in order to determine whether a gene mutation exists.

Additionally, the nucleic acid can be sequenced by any sequencing method known in the art. For example, the viral DNA can be sequenced by the dideoxy method of Sanger et al., 1977, Proc. Natl. Acad. Sci. USA 74:5463, as further described by Messing et al., 1981, Nuc. Acids Res. 9:309, or by the method of Maxam et al., 1980, Methods in Enzymology 65:499. See also the techniques described in Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY.

Antibodies directed against the viral gene products, *i.e.,* viral proteins or viral peptide fragments can also be used to detect mutations in the viral proteins. Alternatively, the viral protein or peptide fragments of interest can be sequenced by any sequencing method known in the art in order to yield the amino acid sequence of the protein of interest. An example of such a method is the Edman degradation method which can be used to sequence small proteins or polypeptides. Larger proteins can be initially cleaved by chemical or enzymatic reagents known in the art, for example, cyanogen bromide, hydroxylamine, trypsin or chymotrypsin, and then sequenced by the Edman degradation method.

### 5.4.2. Correlating Mutations with Resistance to a PI

Any method known in the art can be used to determine whether a mutation is correlated with PI resistance. In one embodiment, univariate analysis is used to identify mutations correlated with PI resistance. Univariate analysis yields P values that indicate the statistical significance of the correlation. In such embodiments, the smaller the P value, the more significant the measurement. Preferably the P values will be less than 0.05. More preferably, P values will be less than 0.01. Even more preferably, the P value will be less than 0.005. P values can be calculated by any means known to one of skill in the art. In one embodiment, P values are calculated using Fisher's Exact Test. In another embodiment, P values can be calculated with Student's t-test. *See, e.g.,* David Freeman, Robert Pisani & Roger Purves, 1980, STATISTICS, W. W. Norton, New York. In certain embodiments, P values can be calculated with both Fisher's Exact Test and Student's t-test. In such embodiments, P values calculated with both tests are preferably less than 0.05. However, a correlation with a P value that is less than 0.10 in one test but less than 0.05 in another test can still be considered to be a marginally significant correlation. Such mutations are suitable for further analysis with, for example, multivariate analysis. Alternatively, further univariate analysis can be performed on a larger sample set to confirm the significance of the correlation.

Further, an odds ratio can be calculated to determine whether a mutation correlates with resistance to a PI. Generally, calculation of odds rations depends on dividing the percentage of virus that comprise a particular mutation or mutations that are identified as resistant to a PI by the percentage of virus with the same mutation or mutations that are identified as not resistant to the PI. In certain embodiments, an odds ratio that is greater than one indicates that the mutation correlates with resistance to a PI. In certain embodiments, an odds ratio that is less than one indicates that the mutation does not correlates with resistance to a PI.

In yet another embodiment, multivariate analysis can be used to determine whether a mutation correlates with PI resistance. Any multivariate analysis known by one of skill in the art to be useful in calculating such a correlation can be used, without limitation. In certain embodiments, a statistically significant number of virus's resistance or susceptibility phenotypes, *e.g.,* IC₅₀, can be determined. These IC₅₀ values can then be divided into groups that correspond to percentiles of the set of IC₅₀ values observed.

After assigning each virus's IC₅₀ value to the appropriate group, the genotype of that virus can be assigned to that group. By performing this method for all viral isolates, the number of instances of a particular mutation in a given percentile of PI susceptibility can be observed. This allows the skilled practitioner to identify mutations that correlate with PI resistance.

Finally, in yet another embodiment, regression analysis can be performed to identify mutations that best predict PI resistance. In such embodiments, regression analysis is performed on a statistically significant number of viral isolates for which genotypes and PI susceptibility phenotypes have been determined. The analysis then identifies which mutations appear to best predict, *e.g.,* most strongly correlate with, PI resistance. Such analysis can then be used to construct rules for predicting PI resistance based upon knowledge of the genotype of a particular virus, described below. In certain embodiments, software such as, for example, CART 5.0, Prism 4.0, SAF, R, or Insightful Miner 3.0 can be used to perform the analysis that identifies the mutations that appear to best predict PI resistance.

### 5.4.3. Computer-Implemented Methods for Determining Resistance to a PI, and Articles Related Thereto

In another aspect, the present invention provides computer-implemented methods for determining whether an HIV-1 is resistant to a PI. In such embodiments, the methods of the invention are adapted to take advantage of the processing power of modern computers. One of skill in the art can readily adapt the methods in such a manner.

Therefore, in certain embodiments, the invention provides a computer-implemented method for determining whether an HIV-1 is resistant to a PI, comprising inputting genetic information into a memory system of a computer, wherein the genetic information indicates whether a mutation in at least one of codon 22, 69, 74, or 83 is present in a gene encoding HIV-1 protease; inputting a correlation between the presence of the mutation and resistance to a PI into the memory system of the computer, and determining whether the HIV-1 is resistant to the PI. In certain embodiments, the genetic information indicates whether a mutation in two, three, or four of codon 22, 69, 74, or 83 is present. In certain embodiments, the genetic information indicates whether a mutation at codon 22 is present. In certain embodiments, the genetic information indicates that the mutation at codon 22 encodes valine (V). In certain embodiments, the genetic information indicates whether a mutation at codon 69 is present. In certain embodiments, the genetic information indicates that the mutation at codon 69 encodes arginine (R). In certain embodiments, the genetic information indicates whether a mutation at codon 74 is present. In certain embodiments, the genetic information indicates that the mutation at codon 74 encodes lysine (K) or serine (S). In certain embodiments, the genetic information indicates whether a mutation at codon 83 is present. In certain embodiments, the genetic information indicates that the mutation at codon 83 encodes aspartic acid (D). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In certain embodiments, the genetic information further indicates whether a mutation in at least one of codon 10, 13, 19, 20, 36, 37, 54, 71, 73, 82, 88, or 93 is present in a gene encoding HIV-1 protease in combination with the mutation in at least one of codon 22, 69, 74, or 83. In certain embodiments, the genetic information indicates whether a mutation in two, three, four, five, six, seven, eight, nine, ten, eleven or twelve of codon 10, 13, 19, 20, 36, 37, 54, 71, 73, 82, 88, or 93 is present in a gene encoding HIV-1 protease. In certain embodiments, the genetic information indicates whether a mutation at codon 10 is present. In certain embodiments, the genetic information indicates whether a mutation at codon 13 is present. In certain embodiments, the genetic information indicates whether a mutation at codon 19 is present. In certain embodiments, the genetic information indicates whether a mutation at codon 20 is present. In certain embodiments, the genetic information indicates whether a mutation at codon 36 is present. In certain embodiments, the genetic information indicates whether a mutation at codon 37 is present In certain embodiments, the genetic information indicates whether a mutation at codon 54 is present. In certain embodiments, the genetic information indicates whether a mutation at codon 71 is present. In certain embodiments, the genetic information indicates whether a mutation at codon 73 is present. In certain embodiments, the genetic information indicates whether a mutation at codon 82 is present. In certain embodiments, the genetic information indicates whether a mutation at codon 88 is present. In certain embodiments, the genetic information indicates whether a mutation at codon 93 is present. In certain embodiments, the genetic information indicates that the mutation at codon 10 encodes isoleucine (I) or valine (V). In certain embodiments, the genetic information indicates that the mutation at codon 13 encodes valine (V). In certain embodiments, the genetic information indicates that the mutation at codon 19 encodes valine (V). In certain embodiments, the genetic information indicates that the mutation at codon 20 encodes isoleucine (I), methionine (M), or threonine (T). In certain embodiments, the genetic information indicates that the mutation at codon 36 encodes isoleucine (I) or valine (V). In certain embodiments, the genetic information indicates that the mutation at codon 37 encodes aspartic acid (D). In certain embodiments, the genetic information indicates that the mutation at codon 54 encodes valine (V). In certain embodiments, the genetic information indicates that the mutation at codon 71 encodes isoleucine (I), threonine (T), or valine (V). In certain embodiments, the genetic information indicates that the mutation at codon 73 encodes serine (S). In certain embodiments, the genetic information indicates that the mutation at codon 82 encodes isoleucine (I). In certain embodiments, the genetic information indicates that the mutation at codon 88 encodes aspartic acid (D). In certain embodiments, the genetic information indicates that the mutation at codon 93 encodes leucine (L). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In certain embodiments, the genetic information further indicates whether a mutation in at least one of codon 23, 24, 30, 32, 46, 47, 48, 50, 54, 82, 84, 88, or 90 is present in a gene encoding HIV-1 protease in combination with the mutation in at least one of codon 22, 69, 74, or 83. In certain embodiments, the genetic information indicates whether a mutation in two, three, four, five, six, seven, eight, nine, ten, eleven, twelve, or thirteen of codon 23, 24, 30, 32, 46, 47, 48, 50, 54, 82, 84, 88, or 90 is present in a gene encoding HIV-1 protease. In certain embodiments, the genetic information indicates whether a mutation at codon 23 is present. In certain embodiments, the genetic information indicates whether a mutation at codon 24 is present. In certain embodiments, the genetic information indicates whether a mutation at codon 30 is present. In certain embodiments, the genetic information indicates whether a mutation at codon 3 is present 2. In certain embodiments, the genetic information indicates whether a mutation at codon 46 is present. In certain embodiments, the genetic information indicates whether a mutation at codon 47 is present. In certain embodiments, the genetic information indicates whether a mutation at codon 48 is present. In certain embodiments, the genetic information indicates whether a mutation at codon 50 is present. In certain embodiments, the genetic information indicates whether a mutation at codon 54 is present. In certain embodiments, the genetic information indicates whether a mutation at codon 82 is present. In certain embodiments, the genetic information indicates whether a mutation at codon 84 is present. In certain embodiments, the genetic information indicates whether a mutation at codon 88 is present. In certain embodiments, the genetic information indicates whether a mutation at codon 90 is present. In certain embodiments, the genetic information indicates that the mutation at codon 23 encodes isoleucine (I). In certain embodiments, the genetic information indicates that the mutation at codon 24 encodes isoleucine (I) or valine (V). In certain embodiments, the genetic information indicates that the mutation at codon 30 encodes asparagine (N). In certain embodiments, the genetic information indicates that the mutation at codon 32 encodes alanine (A) or isoleucine (I). In certain embodiments, the genetic information indicates that the mutation at codon 46 encodes phenylalanine (F), isoleucine (I), leucine (L), or valine (V). In certain embodiments, the genetic information indicates that the mutation at codon 47 encodes alanine (A) or valine (V). In certain embodiments, the genetic information indicates that the mutation at codon 48 encodes alanine (A), glutamic acid (E), leucine (L), methionine (M), or valine (V). In certain embodiments, the genetic information indicates that the mutation at codon 50 encodes leucine (L) or valine (V). In certain embodiments, the genetic information indicates that the mutation at codon 54 encodes alanine (A), leucine (L), serine (S), threonine (T), methionine (M), or valine (V). In certain embodiments, the genetic information indicates that the mutation at codon 82 encodes threonine (T), alanine (A), phenylalanine (F), or serine (S). In certain embodiments, the genetic information indicates that the mutation at codon 84 encodes cysteine (C), alanine (A), or valine (V). In certain embodiments, the genetic information indicates that the mutation at codon 88 encodes serine (S) or threonine (T). In certain embodiments, the genetic information indicates that the mutation at codon 90 encodes methionine (M). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In another aspect, the invention provides a computer-implemented method for determining whether an HIV-1 is resistant to a PI, comprising inputting genetic information into a memory system of a computer, wherein the genetic information indicates whether a mutation in at least one of codon 418 or 482 is present in a gene encoding HIV-1 gag; inputting a correlation between the presence of the mutation and resistance to a PI into the memory system of the computer; and determining whether the HIV-1 is resistant to the PI. In certain embodiments, the genetic information indicates whether a mutation at codon 418 is present. In certain embodiments, the genetic information indicates whether a mutation at codon 482 is present. In certain embodiments, the genetic information indicates whether mutations at codons 418 and 482 are present. In certain embodiments, the genetic information indicates that the mutation at codon 418 encodes glutamic acid (E) or arginine (R). In certain embodiments, the genetic information indicates that the mutation at codon 482 encodes glycine (G). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In certain embodiments, the genetic information further indicates whether a mutation in at least one of codon 431, 437, 449, or 453 is present in a gene encoding HIV-1 gag in combination with the mutation in at least one of codon 418 or 482. In certain embodiments, the genetic information indicates whether a mutation at two, three, or four of codon 431, 437, 449, or 453 is present. In certain embodiments, the genetic information indicates whether a mutation at codon 431 is present. In certain embodiments, the genetic information indicates whether a mutation at codon 437 is present. In certain embodiments, the genetic information indicates whether a mutation at codon 449 is present. In certain embodiments, the genetic information indicates whether a mutation at codon 453 is present. In certain embodiments, the genetic information indicates that the mutation at codon 431 encodes valine (V). In certain embodiments, the genetic information indicates that the mutation at codon 437 encodes valine (V). In certain embodiments, the genetic information indicates that the mutation at codon 449 encodes isoleucine (I) or proline (P). In certain embodiments, the genetic information indicates that the mutation at codon 453 encodes leucine (L). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In another aspect, the invention provides a computer-implemented method for determining whether an HIV-1 is resistant to a PI, comprising inputting genetic information into a memory system of a computer, wherein the genetic information indicates whether a mutation in at least one of codon 418 or 482 is present in a gene encoding HIV-1 gag and/or whether a mutation in at least one of codon 22, 69, 74, or 83 is present in a gene encoding HIV-1 protease; inputting a correlation between the presence of the mutation and resistance to a PI into the memory system of the computer; and determining whether the HIV-1 is resistant to the PI. In certain embodiments, the genetic information further indicates whether a mutation in at least one of codon 431, 437, 449, or 453 is present in a gene encoding HIV-1 gag in combination with the gag and/or protease mutation(s). In certain embodiments, the genetic information further indicates whether a mutation in at least one of codon 23, 24, 30, 32, 46, 47, 48, 50, 54, 82, 84, 88, or 90 is present in a gene encoding HIV-1 protease in combination with the gag and/or protease mutation(s). In certain embodiments, the genetic information further indicates whether a mutation in at least one of codon 10, 13, 19, 20, 36, 37, 54, 71, 73, 82, 88, or 93 is present in a gene encoding HIV-1 protease in combination with the gag and/or protease mutation(s). In certain embodiments, the HIV-1 is resistant to a PI that is AMP, IDV, NFV, RTV, SQV, LPV, or ATV. In certain embodiments, the PI is AMP. In certain embodiments, the PI is IDV. In certain embodiments, the PI is NFV. In certain embodiments, the PI is RTV. In certain embodiments, the PI is SQV. In certain embodiments, the PI is LPV. In certain embodiments, the PI is ATV.

In certain embodiments, the methods further comprise displaying whether the HIV-1 is resistant to a PI on a display of the computer. In certain embodiments, the methods further comprise printing whether the HIV-1 is resistant to a PI.

In another aspect, the invention provides a paper document indicating whether an HIV-1 is resistant to a PI produced according to a method of the invention. In certain embodiments, the paper document is a printed document, *e.g.,* a computer print-out. In still another aspect, the invention provides a computer-readable medium comprising data indicating whether an HIV-1 is resistant to a PI produced according to a method of the invention.

In yet another aspect, the invention provides a computer-readable medium that comprises data indicating whether an HIV-1 is resistant to a PI produced according a method of the invention. In certain embodiments, the computer-readable medium is a random-access memory. In certain embodiments, the computer-readable medium is a fixed disk. In certain embodiments, the computer-readable medium is a floppy disk. In certain embodiments, the computer-readable medium is a portable memory device, such as, *e.g.,* a USB key or an iPod™.

In still another aspect, the invention provides an article of manufacture that comprises computer-readable instructions for performing a method of the invention. In certain embodiments, the article of manufacture is a random-access memory. In certain embodiments, the article of manufacture is a fixed disk. In certain embodiments, the article of manufacture is a floppy disk. In certain embodiments, the article of manufacture is a portable memory device, such as, *e.g.,* a USB key or an iPod™.

In yet another aspect, the invention provides a computer-readable medium that comprises data indicating whether an HIV-1 is resistant to a PI and computer-readable instructions for performing a method of the invention. In certain embodiments, the computer-readable medium is a random-access memory. In certain embodiments, the computer-readable medium is a fixed disk. In certain embodiments, the computer-readable medium is a floppy disk. In certain embodiments, the computer-readable medium is a portable memory device, such as, *e.g.,* a USB key or an iPod™.

In yet another aspect, the invention provides a computer system that is configured to perform a method of the invention.

### 5.4.4. Viruses and Viral Samples

A mutation associated with PI resistance according to the present invention can be present in any type of virus. For example, such mutations may be identified in any virus that infects animals known to one of skill in the art without limitation. In one embodiment of the invention, the virus includes viruses known to infect mammals, including dogs, cats, horses, sheep, cows *etc.* In certain embodiment, the virus is known to infect primates. In preferred embodiments, the virus is known to infect humans. Examples of such viruses that infect humans include, but are not limited to, human immunodeficiency virus ("HIV"), herpes simplex virus, cytomegalovirus virus, varicella zoster virus, other human herpes viruses, influenza A, B and C virus, respiratory syncytial virus, hepatitis A, B and C viruses, rhinovirus, and human papilloma virus. In certain embodiments, the virus is HCV. In other embodiments, the virus is HBV. In a preferred embodiment of the invention, the virus is HIV. Even more preferably, the virus is human immunodeficiency virus type 1 ("HIV-1"). The foregoing are representative of certain viruses for which there is presently available anti-viral chemotherapy and represent the viral families retroviridae, herpesviridae, orthomyxoviridae, paramxyxoviridae, picornaviridae, flaviviridae, pneumoviridae and hepadnaviridae. This invention can be used with other viral infections due to other viruses within these families as well as viral infections arising from viruses in other viral families for which there is or there is not a currently available therapy.

A mutation associated with PI resistance according to the present invention can be found in a viral sample obtained by any means known in the art for obtaining viral samples. Such methods include, but are not limited to, obtaining a viral sample from a human or an animal infected with the virus or obtaining a viral sample from a viral culture. In one embodiment, the viral sample is obtained from a human individual infected with the virus. The viral sample could be obtained from any part of the infected individual's body or any secretion expected to contain the virus. Examples of such parts include, but are not limited to blood, serum, plasma, sputum, lymphatic fluid, semen, vaginal mucus and samples of other bodily fluids. In a preferred embodiment, the sample is a blood, serum or plasma sample.

In another embodiment, a mutation associated with PI resistance according to the present invention is present in a virus that can be obtained from a culture. In some embodiments, the culture can be obtained from a laboratory. In other embodiments, the culture can be obtained from a collection, for example, the American Type Culture Collection.

In certain embodiments, a mutation associated with PI resistance according to the present invention is present in a derivative of a virus. In one embodiment, the derivative of the virus is not itself pathogenic. In another embodiment, the derivative of the virus is a plasmid-based system, wherein replication of the plasmid or of a cell transfected with the plasmid is affected by the presence or absence of the selective pressure, such that mutations are selected that increase resistance to the selective pressure. In some embodiments, the derivative of the virus comprises the nucleic acids or proteins of interest, for example, those nucleic acids or proteins to be targeted by an anti-viral treatment. In one embodiment, the genes of interest can be incorporated into a vector. *See, e.g.,* U.S. Patent Numbers 5,837,464 and 6,242,187 and PCT publication, WO 99/67427, each of which is incorporated herein by reference. In certain embodiments, the genes can be those that encode for a protease or reverse transcriptase.

In another embodiment, the intact virus need not be used. Instead, a part of the virus incorporated into a vector can be used. Preferably that part of the virus is used that is targeted by an anti-viral drug.

In another embodiment, a mutation associated with PI resistance according to the present invention is present in a genetically modified virus. The virus can be genetically modified using any method known in the art for genetically modifying a virus. For example, the virus can be grown for a desired number of generations in a laboratory culture. In one embodiment, no selective pressure is applied (*i.e.,* the virus is not subjected to a treatment that favors the replication of viruses with certain characteristics), and new mutations accumulate through random genetic drift. In another embodiment, a selective pressure is applied to the virus as it is grown in culture (*i.e*., the virus is grown under conditions that favor the replication of viruses having one or more characteristics). In one embodiment, the selective pressure is an anti-viral treatment. Any known anti-viral treatment can be used as the selective pressure.

In certain embodiments, the virus is HIV and the selective pressure is a NNRTI. In another embodiment, the virus is HIV-1 and the selective pressure is a NNRTI. Any NNRTI can be used to apply the selective pressure. Examples of NNRTIs include, but are not limited to, nevirapine, delavirdine and efavirenz. By treating HIV cultured *in vitro* with a NNRTI, one can select for mutant strains of HIV that have an increased resistance to the NNRTI. The stringency of the selective pressure can be manipulated to increase or decrease the survival of viruses not having the selected-for characteristic.

In other embodiments, the virus is HIV and the selective pressure is a NRTI. In another embodiment, the virus is HIV-1 and the selective pressure is a NRTI. Any NRTI can be used to apply the selective pressure. Examples of NRTIs include, but are not limited to, AZT, ddI, ddC, d4T, 3TC, abacavir, and tenofovir. By treating HIV cultured *in vitro* with a NRTI, one can select for mutant strains of HIV that have an increased resistance to the NRTI. The stringency of the selective pressure can be manipulated to increase or decrease the survival of viruses not having the selected-for characteristic.

In still other embodiments, the virus is HIV and the selective pressure is a PI. In another embodiment, the virus is HIV-1 and the selective pressure is a PI. Any PI can be used to apply the selective pressure. Examples of PIs include, but are not limited to, saquinavir, ritonavir, indinavir, nelfinavir, amprenavir, lopinavir and atazanavir. By treating HIV cultured *in vitro* with a PI, one can select for mutant strains of HIV that have an increased resistance to the PI. The stringency of the selective pressure can be manipulated to increase or decrease the survival of viruses not having the selected-for characteristic.

In still other embodiments, the virus is HIV and the selective pressure is an entry inhibitor. In another embodiment, the virus is HIV-1 and the selective pressure is an entry inhibitor. Any entry inhibitor can be used to apply the selective pressure. An example of a entry inhibitor includes, but is not limited to, fusion inhibitors such as, for example, enfuvirtide. Other entry inhibitors include co-receptor inhibitors, such as, for example, AMD3100 (Anormed). Such co-receptor inhibitors can include any compound that interferes with an interaction between HIV and a co-receptor, *e.g.,* CCR5 or CRCX4, without limitation. By treating HIV cultured *in vitro* with an entry inhibitor, one can select for mutant strains of HIV that have an increased resistance to the entry inhibitor. The stringency of the selective pressure can be manipulated to increase or decrease the survival of viruses not having the selected-for characteristic.

In another aspect, a mutation associated with PI resistance according to the present invention can be made by mutagenizing a virus, a viral genome, or a part of a viral genome. Any method of mutagenesis known in the art can be used for this purpose. In certain embodiments, the mutagenesis is essentially random. In certain embodiments, the essentially random mutagenesis is performed by exposing the virus, viral genome or part of the viral genome to a mutagenic treatment. In another embodiment, a gene that encodes a viral protein that is the target of an anti-viral therapy is mutagenized. Examples of essentially random mutagenic treatments include, for example, exposure to mutagenic substances (e.g., ethidium bromide, ethylmethanesulphonate, ethyl nitroso urea (ENU) *etc.*) radiation (e.g., ultraviolet light), the insertion and/or removal of transposable elements (*e.g.,* Tn5, Tn10), or replication in a cell, cell extract, or *in vitro* replication system that has an increased rate of mutagenesis. *See, e.g.,* Russell et al., 1979, Proc. Nat. Acad. Sci. USA 76:5918-5922; Russell, W., 1982, Environmental Mutagens and Carcinogens: Proceedings of the Third International Conference on Environmental Mutagens. One of skill in the art will appreciate that while each of these methods of mutagenesis is essentially random, at a molecular level, each has its own preferred targets.

In another aspect, a mutation associated with PI resistance can be made using site-directed mutagenesis. Any method of site-directed mutagenesis known in the art can be used (*see e.g.,* Sambrook et al., 2001, Molecular Cloning: A Laboratory Manual, Cold Spring Harbor Laboratory, 3rd ed., NY; and Ausubel et al., 1989, Current Protocols in Molecular Biology, Greene Publishing Associates and Wiley Interscience, NY). *See, e.g.,* Sarkar and Sommer, 1990, Biotechniques, 8:404-407. The site directed mutagenesis can be directed to, *e.g.,* a particular gene or genomic region, a particular part of a gene or genomic region, or one or a few particular nucleotides within a gene or genomic region. In one embodiment, the site directed mutagenesis is directed to a viral genomic region, gene, gene fragment, or nucleotide based on one or more criteria. In one embodiment, a gene or a portion of a gene is subjected to site-directed mutagenesis because it encodes a protein that is known or suspected to be a target of an anti-viral therapy, *e.g.,* the gene encoding the HIV protease. In another embodiment, a portion of a gene, or one or a few nucleotides within a gene, are selected for site-directed mutagenesis. In one embodiment, the nucleotides to be mutagenized encode amino acid residues that are known or suspected to interact with an anti-viral compound. In another embodiment, the nucleotides to be mutagenized encode amino acid residues that are known or suspected to be mutated in viral strains that are resistant or susceptible or resistant to one or more antiviral agents. In another embodiment, the mutagenized nucleotides encode amino acid residues that are adj acent to or near in the primary sequence of the protein residues known or suspected to interact with an anti-viral compound or known or suspected to be mutated in viral strains that are resistant or susceptible or resistant to one or more antiviral agents. In another embodiment, the mutagenized nucleotides encode amino acid residues that are adjacent to or near to in the secondary, tertiary or quaternary structure of the protein residues known or suspected to interact with an anti-viral compound or known or suspected to be mutated in viral strains having an altered replication capacity. In another embodiment, the mutagenized nucleotides encode amino acid residues in or near the active site of a protein that is known or suspected to bind to an anti-viral compound.

### 6. EXAMPLES

### 6.1. Example 1: Measuring PI Resistance Using Resistance Test Vectors

This example provides methods and compositions for accurately and reproducibly measuring the resistance or sensitivity of HIV-1 to antiretroviral drugs including, for example, PIs such as AMP, IDV, NFV, RTV, SQV, LPV, and/or ATV. The methods for measuring resistance or susceptibility to such drugs can be adapted to other HIV strains, such as HIV-2, or to other viruses, including, but not limited to hepadnaviruses (*e.g.,* human hepatitis B virus), flaviviruses (*e.g.,* human hepatitis C virus) and herpesviruses (*e.g*., human cytomegalovirus).

Drug resistance tests can be carried out, for example, using the methods for phenotypic drug susceptibility and resistance tests described in US Patent Number 5,837,464 (International Publication Number WO 97/27319) which is hereby incorporated by reference in its entirety, or according to the protocol that follows.

Patient-derived segment(s) corresponding to the HIV protease and reverse transcriptase coding regions were amplified by the reverse transcription-polymerase chain reaction method (RT-PCR) using viral RNA isolated from viral particles present in the plasma or serum of HIV-infected individuals as follows. Viral RNA was isolated from the plasma or serum using oligo-dT magnetic beads (Dynal Biotech, Oslo, Norway), followed by washing and elution of viral RNA. The RT-PCR protocol was divided into two steps. A retroviral reverse transcriptase (e.g. Moloney MuLV reverse transcriptase (Roche Molecular Systems, Inc., Branchburg, NJ; Invitrogen, Carlsbad, CA), or avian myeloblastosis virus (AMV) reverse transcriptase (Roche Molecular Systems, Inc., Branchburg, NJ), or) was used to copy viral RNA into cDNA. The cDNA was then amplified using a thermostable DNA polymerase (*e.g. Taq* (Roche Molecular Systems, Inc., Branchburg, NJ), *Tth* (Roche Molecular Systems, Inc., Branchburg, NJ), PRIMEZYME™ (isolated from *Thermus brockianus,* Biometra, Gottingen, Germany)) or a combination of thermostable polymerases as described for the performance of "long PCR" (Barnes, W.M., 1994, Proc. Natl. Acad. Sci, USA 91, 2216-20) (e.g. Expand High Fidelity PCR System (Taq + Pwo), (Boehringer Mannheim. Indianapolis, IN); GENEAMP XL™ PCR kit (*Tth* + Vent), (Roche Molecular Systems, Inc., Branchburg, NJ); or ADVANTAGE II®, Clontech, Palo Alto, CA.)

PCR primers were designed to introduce *ApaI* and *PinA1* recognition sites into the 5' or 3' end of the PCR product, respectively.

Resistance test vectors incorporating the "test" patient-derived segments were constructed as described in US Patent Number 5,837,464 using an amplified DNA product of 1.5 kB prepared by RT-PCR using viral RNA as a template and oligonucleotides PDS Apa, PDS Age, PDS PCR6, Apa-gen, Apa-c, Apa-f, Age-gen, Age-a, RT-ad, RT-b, RT-c, RT-f, and/or RT-g as primers, followed by digestion with *Apa*I and *Age*I or the isoschizomer *Pin*A1*.* To ensure that the plasmid DNA corresponding to the resultant fitness test vector comprises a representative sample of the HIV viral quasi-species present in the serum of a given patient, many (>250) independent *E. coli* transformants obtained in the construction of a given fitness test vector are pooled and used for the preparation of plasmid DNA.

A packaging expression vector encoding an amphotrophic MuLV 4070A *env* gene product enables production in a resistance test vector host cell of resistance test vector viral particles which can efficiently infect human target cells. Resistance test vectors encoding all HIV genes with the exception of env were used to transfect a packaging host cell (once transfected the host cell is referred to as a fitness test vector host cell). The packaging expression vector which encodes the amphotrophic MuLV 4070A *env* gene product is used with the resistance test vector to enable production in the resistance test vector host cell of infectious pseudotyped resistance test vector viral particles.

Drug resistance tests performed with resistance test vectors were carried out using packaging host and target host cells consisting of the human embryonic kidney cell line 293. See U.S. Patent No 5,837,464.

Resistance tests were carried out with resistance test vectors using two host cell types. Resistance test vector viral particles were produced by a first host cell (the resistance test vector host cell) that was prepared by transfecting a packaging host cell with the resistance test vector and the packaging expression vector. The resistance test vector viral particles were then used to infect a second host cell (the target host cell) in which the expression of the indicator gene is measured.

The resistance test vectors containing a functional luciferase gene cassette were constructed as described above and host cells were transfected with the resistance test vector DNA. The resistance test vectors contained patient-derived reverse transcriptase and protease DNA sequences that encode proteins which were either susceptible or resistant to the antiretroviral agents, such as, for example, NRTIs, NNRTIs, and PIs.

The amount of luciferase activity detected in infected cells is used as a direct measure of "infectivity," *i.e.,* the ability of the virus to complete a single round of replication. Thus, drug resistance or sensitivity can be determined by plotting the amount of luciferase activity produced by patient derived viruses in the presence of varying concentrations of the antiviral drug. By identifying the concentration of drug at which luciferase activity is half of that observed in the absence of drug, the IC₅₀ of the virus from which patient-derived segment(s) were obtained for the antiretroviral agent can be determined.

Host (293) cells were seeded in 10-cm-diameter dishes and were transfected one day after plating with resistance test vector plasmid DNA and the envelope expression vector. Transfections were performed using a calcium-phosphate co-precipitation procedure. The cell culture media containing the DNA precipitate was replaced with fresh medium, from one to 24 hours, after transfection. Cell culture medium containing resistance test vector viral particles was harvested one to four days after transfection and was passed through a 0.45-mm filter before optional storage at -80 °C. Before infection, target cells (293 cells) were plated in cell culture media. Control infections were performed using cell culture media from mock transfections (no DNA) or transfections containing the resistance test vector plasmid DNA without the envelope expression plasmid. One to three or more days after infection the media was removed and cell lysis buffer (Promega Corp.; Madison, WI) was added to each well. Cell lysates were assayed for luciferase activity. Alternatively, cells were lysed and luciferase was measured by adding Steady-Glo (Promega Corp.; Madison, WI) reagent directly to each well without aspirating the culture media from the well. The amount of luciferase activity produced in infected cells was normalized to adjust for variation in transfection efficiency in the transfected host cells by measuring the luciferase activity in the transfected cells, which is not dependent on viral gene functions, and adjusting the luciferase activity from infected cell accordingly.

### 6.2. Example 2: Identifying Mutations Correlated with Resistance to a PI

This example provides methods and compositions for identifying mutations that correlate with resistance to a PI. Resistance test vectors were constructed and used as described in Example 1. Resistance test vectors derived from patient samples or clones derived from the resistance test vector pools were tested in a resistance assay to determine accurately and quantitatively the relative AMP, IDV, NFV, RTV, SQV, LPV, or ATV resistance or susceptibility compared to the median observed resistance or susceptibility.

### Genotypic analysis of patient HIV samples:

Resistance test vector DNAs, either pools or clones, can be analyzed by any genotyping method, e.g., as described above. In this example, patient HIV sample sequences were determined using viral RNA purification, RT/PCR and ABI chain terminator automated sequencing. The sequence that was determined was compared to that of a reference sequence, NL4-3. The genotype was examined for sequences that were different from the reference or pre-treatment sequence and correlated to the observed IC₅₀ for AMP, IDV, NFV, RTV, SQV, LPV, and ATV.

### Correlation of Mutations with Resistance to a PI:

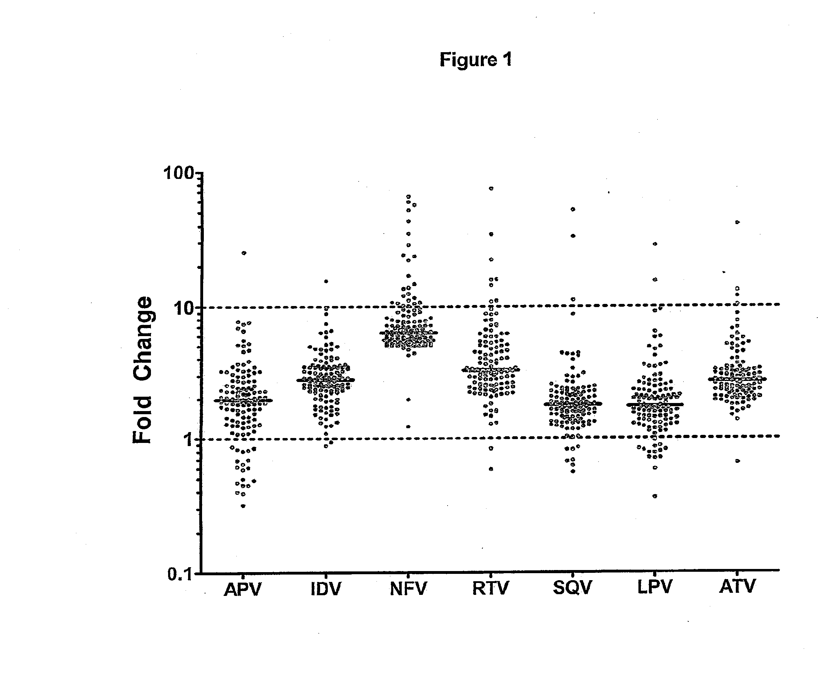

To identify heretofore unrecognized mutations associated with PI resistance, a dataset of 45,528 clinical HIV-1 samples subjected to phenotypic and genotypic assays was screened with two statistical methods. First, a dataset was constructed to remove mutations known to be correlated with PI resistance. Thus, clinical samples that have one or more primary PI-selected resistance-associated mutations (RAMs) were excluded from the dataset. Primary PI RAMs were defined as any change vs. wild-type (HIV-1 strain NL4-3) at positions 23, 24, 30, 32, 46, 47, 48, 50, 54, 82, 84, 88, 90, with the exception of I54V, N88D, and V82I. Following this exclusion, 27,164 clinical samples were identified with no primary PI RAMs according to this definition. Of these samples, 1360 were identified that exhibited a fold-change (FC) in IC₅₀ for a PI above a biological cutoff; the 1360 samples exhibited a FC that is in the 99^{th} percentile of FCs observed for a statistically-significant number of viral isolates. Of these 1360 samples, 141 were identified that exhibited an FC in IC₅₀ for a PI greater than five. Of these 141 samples, 125 were unique samples from individual patients. 28 of these samples exhibited an FC in IC₅₀ for at least one PI greater than ten. The FC in IC₅₀ observed for the 125 samples are summarized in Table 1, below, while a scatter diagram presenting the FC in IC₅₀ observed for the 125 samples for the PIs is presented as Figure 1.

**Table 1**

| | **AMP** | **IDV** | **NFV** | **RTV** | **SQV** | **LPV** | **ATV** |
|---|---|---|---|---|---|---|---|
| **mean** | 2.4 | 3.1 | 9.6 | 5.1 | 2.7 | 2.4 | 3.6 |
| **median** | 2.0 | 2.8 | 6.3 | 3.3 | 1.8 | 1.8 | 2.7 |
| **min** | 0.3 | 0.9 | 1.2 | 0.6 | 0.6 | 0.4 | 0.7 |
| **max** | 25.4 | 15.5 | 65.1 | 74.6 | 52.0 | 28.4 | 40.7 |
| **count** | 125 | 125 | 125 | 125 | 125 | 119 | 113 |

Table 2, below, shows the number of individual samples resistant to 1, 2, 3, 4, 5, 6, or all 7 of AMP, IDV, NFV, RTV, SQV, LPV, and ATV, measured by an FC in IC₅₀ greater than the Biological Cutoff, greater than 5 and greater than 10. Of the samples resistant to only 1 PI, 85 of 89 were resistant to NFV. Of the 17 samples resistant to two PIs, 12 were resistant to NFV and RTV.

**Table 2**

| | **Number of Samples with FC:** | | |
|---|---|---|---|
| **Number of PIs** | **> Biological Cutoff** | **> 5** | **>10** |
| **1** | **6 (4.8)** | **89 (71.2)** | **20 (16.0)** |
| **2** | **12 (9.6)** | **17(13.6)** | **5(4.0)** |
| **3** | **17 (13.6)** | **11 (8.8)** | **1 (0.8)** |
| **4** | **20 (16.0)** | **3 (2.4)** | **1(0.8)** |
| **5** | **12 (9.6)** | **1 (0.8)** | **0(0.0)** |
| **6** | **29 (23.2)** | **2(1.6)** | **1 (0.8)** |
| **7** | **29(23.2)** | **2(1.6)** | **0(0.0)** |

In the first statistical analysis, Fisher's exact test was used to identify genotypic changes in gag or PR associated with reduced PI susceptibility; mixtures were counted as mutant, and variables with p values less than 0.005 were considered significant. Compared to 3956 samples with no primary PI RAMs and an FC in IC₅₀ for all PIs less than 5, PR mutations over-represented in samples with FC in IC₅₀ for at least 1 PI that is greater than 5 included L10IV, I13V, L19V, K20IMT, A22V, M36IV, N37D, 154V, H69R, A71ITV, G73S, T74KS, V82I, N83D, N88D and I93L. In gag, several changes including K418ER, A431V, I437V, L449IP, P453L, and E482G were significantly associated with FC in IC₅₀ for at least 1 PI greater than 5. The protease mutations identified as correlated with PI resistance with Fischer's exact test are presented in Table 3, below. In Table 3, any non-wild-type amino acid present at a particular codon is represented with a blank, e.g., I54 represents samples where the amino acid at codon 54 is any amino acid other than isoleucine (I). In Table 3, mt S represents the percentage of samples with the mutation that were phenotypically sensitive to a protease, mt R represents the percentage of samples with the mutation that were phenotypically resistant to a protease, and R/S indicates the percentage of resistant samples divided by the percentage of sensitive samples.

**Table 3**

| **mutation** | **n** | **P value** | **mt S (%)** | **mt R (%)** | **R/S** |
|---|---|---|---|---|---|
| **L89I** | **6** | **<0.0001** | **0.0%** | **4.8%** | **>>>** |
| **K20A** | **2** | **0.0009** | **0.0%** | **1.6%** | **>>>** |
| **T74P** | **2** | **0.0009** | **0.0%** | **1.6%** | **>>>** |
| **A22V** | **8** | **<0.0001** | **0.03%** | **5.6%** | **221.5** |
| **I54** | **20** | **<0.0001** | **0.1%** | **12.0%** | **94.9** |
| **I54V** | **20** | **<0.0001** | **0.1%** | **12.0%** | **94.9** |
| **E35G** | **3** | **0.0027** | **0.03%** | **1.6%** | **63.3** |
| **L76V** | **3** | **0.0027** | **0.03%** | **1.6%** | **63.3** |
| **L89V** | **3** | **0.0027** | **0.03%** | **1.6%** | **63.3** |
| **K20T** | **30** | **<0.0001** | **0.3%** | **15.2%** | **54.7** |
| **N88D** | **13** | **<0.0001** | **0.1%** | **6.4%** | **50.6** |
| **K20V** | **5** | **0.0003** | **0.1%** | **2.4%** | **47.5** |
| **N83D** | **5** | **0.0003** | **0.1%** | **2.4%** | **47.5** |
| **T74S** | **51** | **<0.0001** | **0.7%** | **20.0%** | **30.4** |
| **A71I** | **13** | **<0.0001** | **0.2%** | **4.8%** | **27.1** |
| **T74K** | **12** | **<0.0001** | **0.2%** | **4.0%** | **22.6** |
| **G73S** | **11** | **0.0002** | **0.2%** | **3.2%** | **18.1** |
| **E35N** | **9** | **0.0021** | **0.2%** | **2.4%** | **15.8** |
| **K20I** | **45** | **<0.0001** | **0.8%** | **9.6%** | **11.5** |
| **H69R** | **20** | **0.0003** | **- 0.4%** | **4.0%** | **10.5** |
| **M36V** | **21** | **0.0003** | **0.4%** | **4.0%** | **9.9** |
| **K20** | **260** | **<0.0001** | **5.5%** | **35.2%** | **6.4** |
| **Q92K** | **33** | **0.0030** | **0.7%** | **4.0%** | **5.7** |
| **V82I** | **91** | **<0.0001** | **2.0%** | **10.4%** | **5.3** |
| **K20M** | **36** | **0.0044** | **0.8%** | **4.0%** | **5.1** |
| **A71V** | **175** | **<0.0001** | **3.8%** | **19.2%** | **5.0** |
| **L19V** | **65** | **0.0007** | **1.4%** | **6.4%** | **4.4** |
| **A71** | **485** | **<0.0001** | **10.9%** | **43.2%** | **4.0** |
| **L10V** | **181** | **<0.0001** | **4.1%** | **15.2%** | **3.7** |
| **A71T** | **318** | **<0.0001** | **7.2%** | **26.4%** | **3.7** |
| **H69K** | **79** | **0.0026** | **1.8%** | **6.4%** | **3.6** |
| **L89** | **110** | **0.0005** | **2.5%** | **8.8%** | **3.5** |
| **L10** | **532** | **<0.0001** | **12.1%** | **42.4%** | **3.5** |
| **L10I** | **349** | **<0.0001** | **8.0%** | **27.2%** | **3.4** |
| **M36** | **984** | **<0.0001** | **23.0%** | **59.2%** | **2.6** |
| **M36I** | **936** | **<0.0001** | **21.9%** | **54.4%** | **2.5** |
| **I13V** | **886** | **<0.0001** | **20.9%** | **47.2%** | **2.3** |
| **I93L** | **1083** | **<0.0001** | **25.6%** | **57.6%** | **2.3** |
| **N37D** | **519** | **<0.0001** | **12.3%** | **25.6%** | **2.1** |
| **162V** | **1066** | **<0.0001** | **25.4%** | **49.6%** | **2.0** |
| **E35D** | **1072** | **<0.0001** | **25.6%** | **48.0%** | **1.9** |

The gag mutations identified as correlated with PI resistance with Fischer's exact test are presented in Table 4, below. In Table 4, any non-wild-type amino acid present at a particular codon is represented with a blank, e.g., A431 represents samples where the amino acid at codon 431 is any amino acid other than alanine (A). ). In Table 4, mt S represents the percentage of samples with the mutation that were phenotypically sensitive to a protease, mt R represents the percentage of samples with the mutation that were phenotypically resistant to a protease, and R/S indicates the percentage of resistant samples divided by the percentage of sensitive samples.

**Table 4**

| **mutation** | **n** | **P value** | **mt S (%)** | **mt R (%)** | **R/S** |
|---|---|---|---|---|---|
| **A431V** | **17** | **<0.0001** | **0.2%** | **8.9%** | **58.5** |
| **A431** | **25** | **<0.0001** | **0.3%** | **9.7%** | **29.4** |
| **K418E** | **11** | **0.0038** | **0.2%** | **2.4%** | **12.0** |
| **I437V** | **91** | **<0.0001** | **1.7%** | **17.7%** | **10.2** |
| **I437** | **176** | **<0.0001** | **3.8%** | **20.2%** | **5.3** |
| **L449I** | **36** | **0.0042** | **0.8%** | **14.0%** | **5.1** |
| **E482G** | **110** | **0.0017** | **2.5%** | **8.1%** | **3.2** |
| **E467** | **119** | **0.0030** | **2.8%** | **8.1%** | **2.9** |
| **Q474** | **123** | **0.0038** | **2.9%** | **8.1%** | **2.8** |
| **P453L** | **229** | **0.0002** | **5.3%** | **14.5%** | **2.7** |
| **K418R** | **527** | **<0.0001** | **12.3%** | **31.5%** | **2.5** |
| **L449** | **413** | **<0.0001** | **9.7%** | **24.2%** | **2.5** |
| **K418** | **850** | **<0.0001** | **20.1%** | **43.5%** | **2.2** |
| **P453** | **405** | **0.0011** | **9.6%** | **19.4%** | **2.0** |
| **L449P** | **339** | **0.0041** | **8.1%** | **16.1%** | **2.0** |
| **S499deI** | **366** | **0.0036** | **8.7%** | **16.9%** | **1.9** |
| **E428K** | **2** | **0.0009** | **0.0%** | **1.6%** | **0.0** |

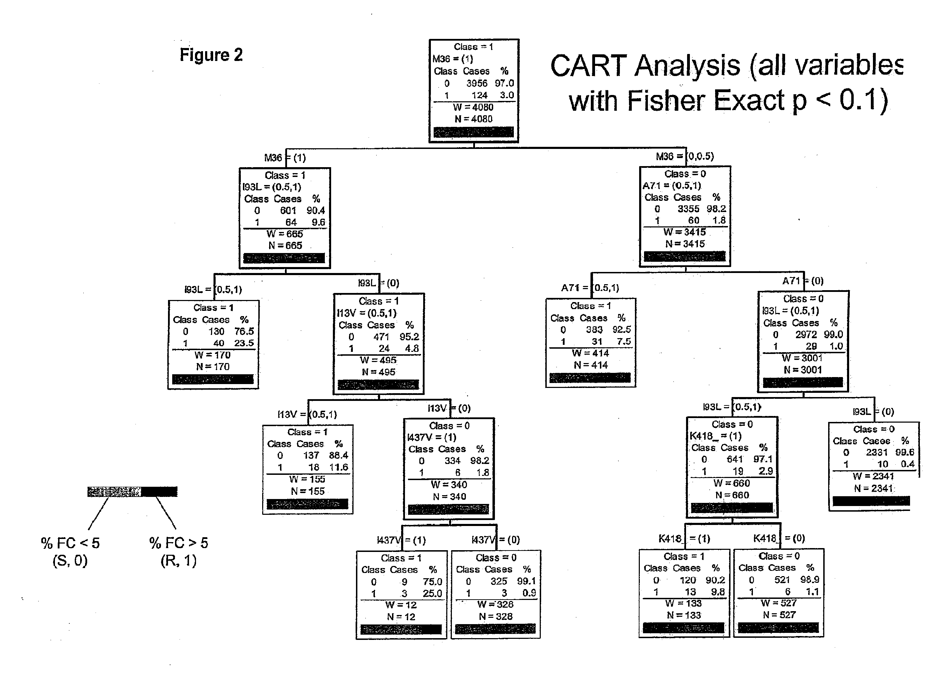

In the second analysis, Classification and Regression Tree (CART 5.0; Salford Systems, San Diego, CA) analysis was used to identify mutations that best segregated samples exhibiting a FC in the IC₅₀ of at least one PI greater than five from those with a FC less than five for all PIs. Such samples with a FC greater than five were scored as resistant. A total of 4080 samples with no primary PI RAMs were considered, 3956 with FC less than five and 124 with FC greater than five. The classification tree generated in the analysis is shown as Figure 2. In Figure 2, the presence of a particular mutation is indicated by the notation, e.g., M36 = (1); the absence of a particular mutation is indicated by the notation, e.g., M36 = (0); and the presence of any non-wild-type amino acid at a particular codon is indicated by the notation, *e.g.,* M36 = 0.5.

As shown in Figure 2, the protease mutation M36I or M36V is the first segregator of patient samples. Of the 4080 total samples, 665 had either M36I or M36V, while 3415 samples did not comprise either mutation or the mutations were present only as mixtures with wild-type. Of the samples with M36I or M36V, 64 (9.6%) were resistant to at least one PI, while only 1.8% of samples without one of these mutations or with a mixture were resistant to a PI.

Of samples with M36I or M36V, 170 also contained at least a mixture of I93L in protease, of which 40 (23.5%) were resistant to at least 1 PI. Only 4.8% of M36I or M36V-containing samples were resistant to a PI in the absence of I93L. However, of these samples comprising M36I or M36V but not I93L, 155 comprised I13V in protease, of which 18 (11.6%) were resistant to at least one PI. Of 340 samples with M36I or.M36V but not I93L or I13V, 12 comprised the gag mutation I437V of which 3 (25%) were resistant to at least one protease. 328 samples comprised M36I or M36V but not I93L, I13V, or I437V, of which 325 (99.1%) were not resistant to any tested PI.

Of 3415 samples without M36I or M36V either alone or in mixture with wild-type, only 60 (1.8%) were resistant to at least one PI. Of these samples, 414 comprised A71I, A71T, or A71V in protease either alone or in mixture with wild-type, of which 31 (7.5%) were resistant to a PI. Only 29 of 3001 samples without M36I or M36V and A71I, A71T, or A71V were resistant to at least one PI. Of these 3001 samples, 660 comprised I93L in protease either alone or in mixture with wild-type. 19 of these 660 samples were resistant to a PI, while only 10 of 2341 samples without I93L were resistant to a PI. Of the samples with I93L in the absence of M36I or M36V and A71I, A71T, or A71V, 133 comprised K418E or K418R in gag, of which 13 (9.8%) were resistant to a PL

### 6.3. Example 3: A Representative Patient Sample

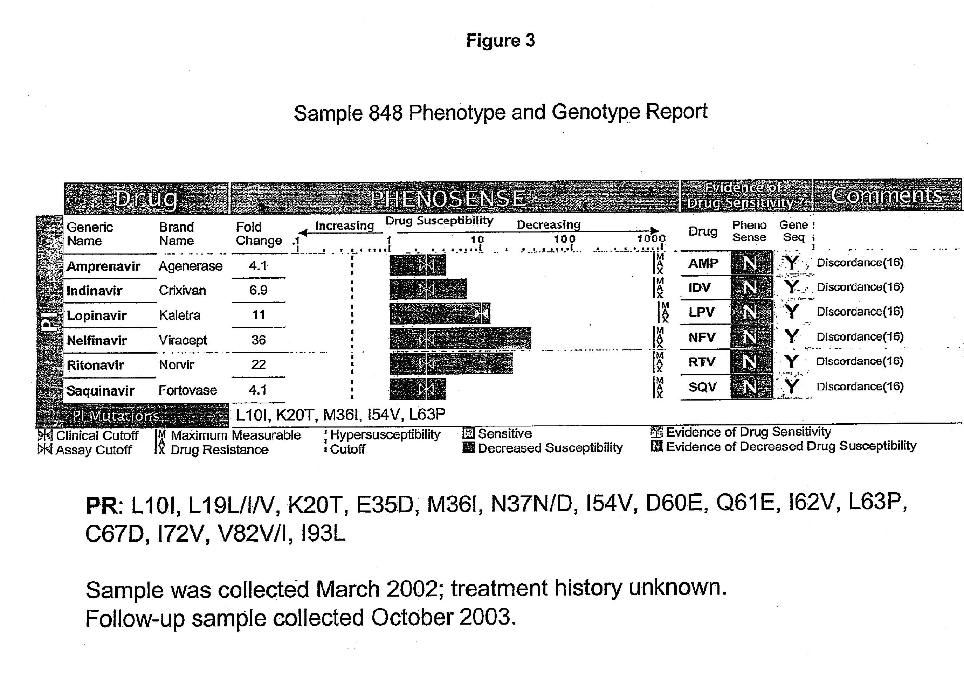

This example describes the results of phenotypic assays for viral samples isolated from an individual patient sample, sample 848. Genotypic analysis of the patient sample revealed that the patient's protease comprised L10I, K20T, B35D, M36I, N37N/D, I54V, D60E, Q61E, I62V, L63P, C67D, I72V, and I93L and mixtures at codon 19 of L19L, L19I, and L19V and at codon 82 of V82V and V82I. The results of phenotypic analysis of the mixed viral population in sample 848 are presented as Figure 3. As shown in Figure 3, the viral population in sample 848 was scored as resistant to all tested PIs, including AMP, IDV, LPV, NFV, RTV, and SQV.

In addition, the changes in the viral populations genotype was monitored over time, as shown in Table 5, below.

**Table 5**

| **DATE** | **PR MUTATIONS** | **APV** | **IDV** | **NFV** | **RTV** | **SQV** | **LPV** | **ATV** |
|---|---|---|---|---|---|---|---|---|
| March 2002 | L10I, L19L/I/V, K20T, E35D, M36I, N37N/D, 154V, D60E, Q61E, 162V, L63P, C67D, I72V, V82V/I, I93L | 4.1 | 6.9 | 35.6 | 21.6 | 4.1 | 10.6 | nt |
| October 2003 | L10I, L19I/V, K20T, E35D, M36I, N37N/D, I54V, D60E, Q61E, I62V, L63P, C67D, I72V, V82V/I, I93L | 6.6 | 9.8 | 52.0 | 34.2 | 11.2 | 9.5 | 10.4 |

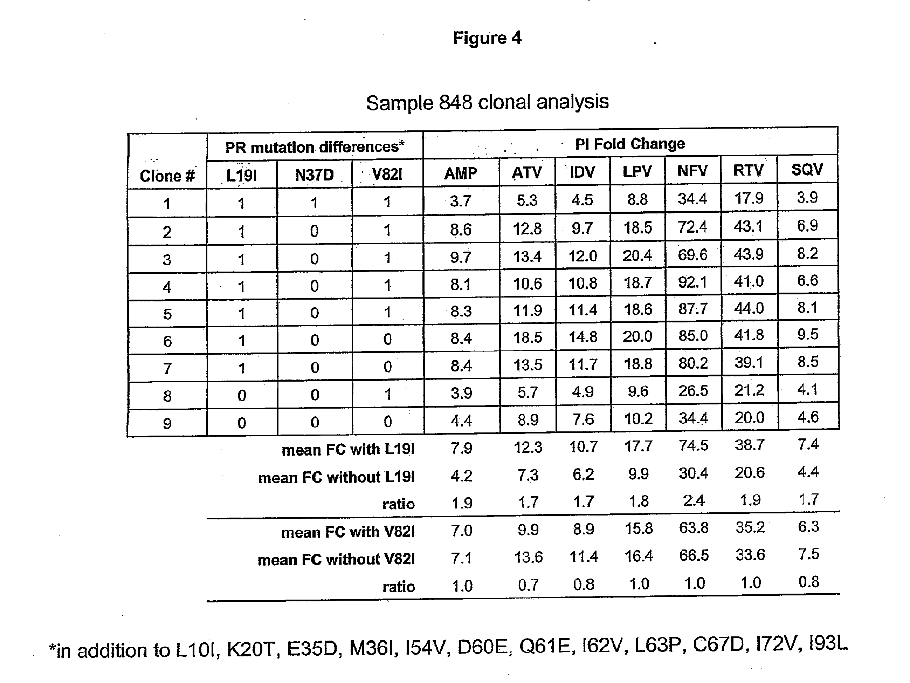

To dissect the relative contributions of the mixed mutations, individual test vectors were isolated from the population representing sample 848 and the phenotypes of the different mixtures were assessed. In particular, the PI resistance phenotypes of sample 848 were tested in the presence and absence of L19I and V82I. Results from this analysis are presented as Figure 4. In brief, V82I showed no significant effect on resistance to any PI, while the presence of L19I in the genotypic background of sample 848 resulted in an approximately two-fold increase to FC for all tested PIs.

Finally, the C-terminal region of gag was sequenced to determine the gag genotype of sample 848. Sample 848 comprised mutations at gag codons 436, 437, 449, 453, 471, 479, and 487.

### 6.4. Example 4: Analysis of Variability in PI Susceptibility for Resistant Viruses

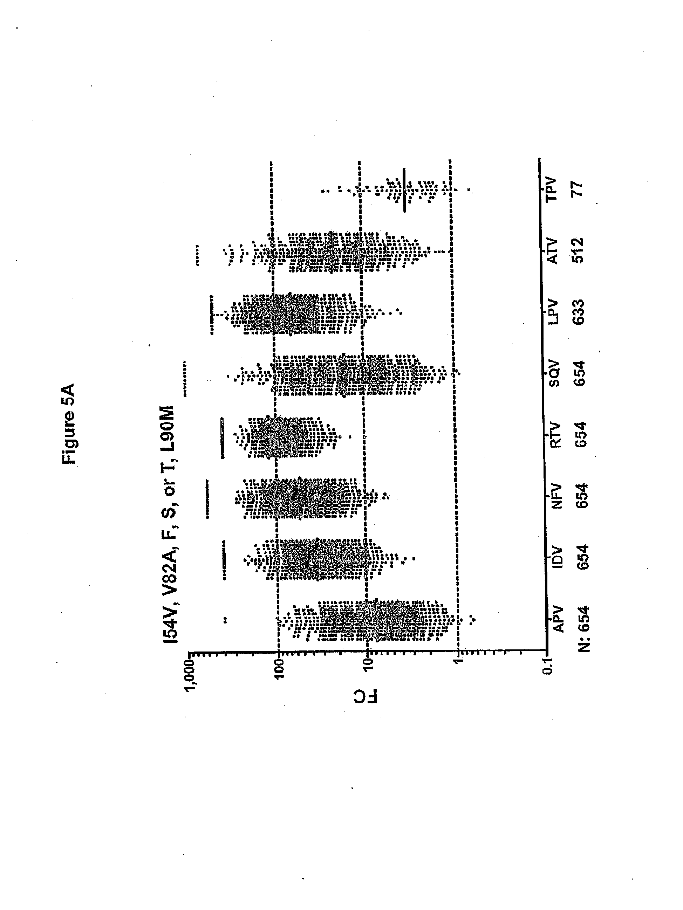

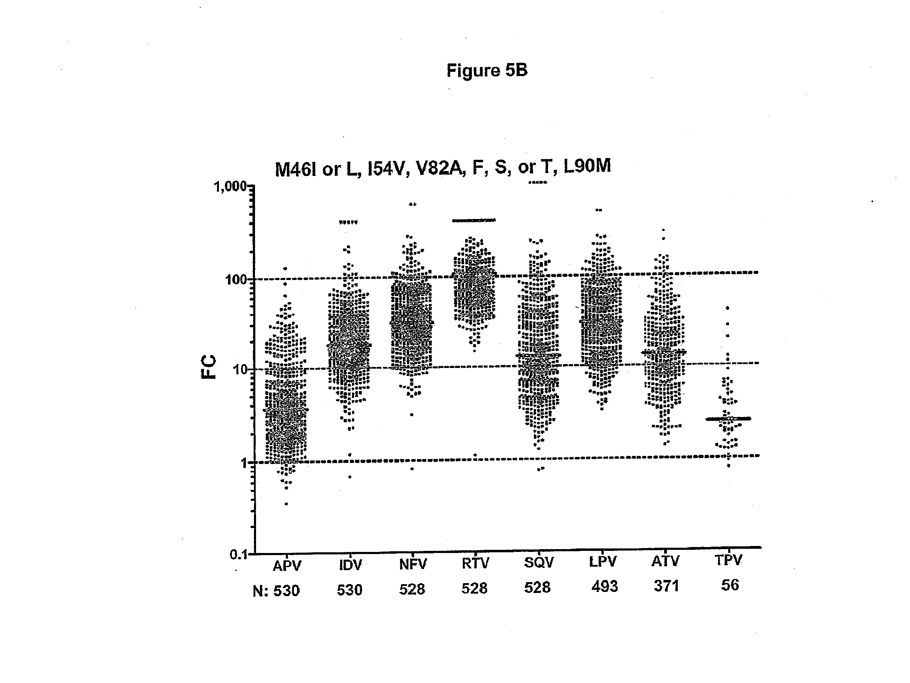

This example describes analysis of variations in susceptibility to PIs among resistant viruses due to mutations in gag in the presence of recognized primary PI mutations. First, to make an initial assessment of the possibility that changes in the gag region also modulate the PI susceptibility in PI resistant samples, two groups of samples were defined based on the presence or absence of primary PI mutations I54V, V82A, F, S, or T, and L90M, with or without M46I or L. Samples with mutations at positions 24, 30, 32, 47, 48, 50, 84, 88 were excluded, and the number of secondary mutations in the two groups was variable. The distribution in susceptibility of the two groups is presented in the scatter diagrams of Figures 5A and 5B. Extensive variability in PR fold change was observed within each group of samples, indicating that mutations other than the primary mutations are important for determining susceptibility, *i.e.,* secondary PI mutations and/or gag mutations.

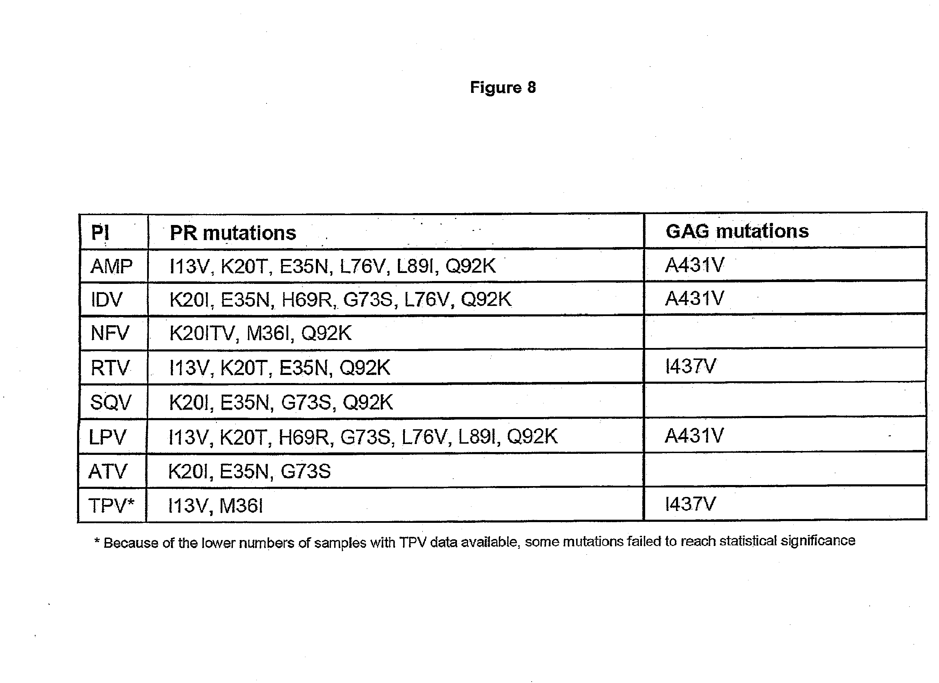

Next, all samples with gag sequences available from both of the above groups of samples were combined (n=502) and the ratio of each PI fold change in resistance value to the median within each group was calculated. The mutations, the number of times each mutation was observed in the sample set, the percentage of samples that had the mutation and were scored as resistant (mtR) or susceptible (mtR), and the significance of the correlation between the presence of the mutation and the resistant phenotype are each presented in Figures 6A and B (gag mutations) and 7A-H (protease mutations). Mutations in protease or gag which are associated with PI FC over (R/S ratio>1) or under (R/S ratio < 1) the median in these primary PI mutation-containing samples were compared to the list of mutations described in Tables 3 and 4, above, for the unexplained PI resistant samples; the mutations in common are listed in Figure 8.

All references cited herein are incorporated by reference in their entireties.

The examples provided herein, both actual and prophetic, are merely embodiments of the present invention and are not intended to limit the invention in any way.

Preferred embodiments:
1. A method for determining whether an HIV-1 is resistant to a PI, comprising detecting whether a mutation in at least one of codon 22, 69, 74, or 83 is present in a gene encoding protease of the HIV-1, wherein the presence of the mutation correlates with resistance to a PI, thereby determining whether the HIV-1 is resistant to the PI.
2. The method of item 1, further comprising detecting whether a mutation in at least one of codon 10, 13, 19, 20, 36, 37, 54, 71, 73, 82, 88, or 93 is present in the gene encoding protease of the HIV-1 in combination with the mutation in at least one of codon 22, 69, 74, or 83, wherein the presence of the mutations correlates with resistance to a PI, thereby determining whether the HIV-1 is resistant to the PI.
3. The method of item 1, further comprising detecting whether a mutation in at least one of codon 23, 24, 30, 32, 46, 47, 48, 50, 54, 82, 84, 88, or 90 is present in the gene encoding protease of the HIV-1 in combination with the mutation in at least one of codon 22, 69, 74, or 83, wherein the presence of the mutations correlates with resistance to a PI, thereby determining whether the HIV-1 is resistant to the PI.
4. The method of item 1, 2, or 3, wherein the mutation at codon 22 encodes valine (V).
5. The method of item 1, 2, or 3, wherein the mutation at codon 69 encodes arginine (R).
6. The method of item 1, 2, or 3, wherein the mutation at codon 74 encodes lysine (K) or serine (S).
7. The method of item 1, 2, or 3, wherein the mutation at codon 83 encodes aspartic acid (D).
8. The method of item 2, wherein the mutation at codon 10 encodes isoleucine (I) or valine (V).
9. The method of item 2, wherein the mutation at codon 13 encodes valine (V).
10. The method of item 2, wherein the mutation at codon 19 encodes valine (V).
11. The method of item 2, wherein the mutation at codon 20 encodes isoleucine (I), methionine (M), or threonine (T).
12. The method of item 2, wherein the mutation at codon 36 encodes isoleucine (I) or valine (V).
13. The method of item 2, wherein the mutation at codon 37 encodes aspartic acid (D).
14. The method of item 2, wherein the mutation at codon 54 encodes valine (V).
15. The method of item 2, wherein the mutation at codon 71 encodes isoleucine (I), threonine (T), or valine (V).
16. The method of item 2, wherein the mutation at codon 73 encodes serine (S).
17. The method of item 2, wherein the mutation at codon 82 encodes isoleucine (I).
18. The method of item 2, wherein the mutation at codon 88 encodes aspartic acid (D).
19. The method of item 2, wherein the mutation at codon 93 encodes leucine (L).
20. The method of item 3, wherein the mutation at codon 23 encodes isoleucine (I).
21. The method of item 3, wherein the mutation at codon 24 encodes isoleucine (I) or valine (V).
22. The method of item 3, wherein the mutation at codon 30 encodes asparagine (N).
23. The method of item 3, wherein the mutation at codon 32 encodes alanine (A) or isoleucine (I).
24. The method of item 3, wherein the mutation at codon 46 encodes phenylalanine (F), isoleucine (I), leucine (L), or valine (V).
25. The method of item 3, wherein the mutation at codon 47 encodes alanine (A) or valine (V).
26. The method of item 3, wherein the mutation at codon 48 encodes alanine (A), glutamic acid (E), leucine (L), methionine (M), or valine (V).
27. The method of item 3, wherein the mutation at codon 50 encodes leucine (L) or valine (V).
28. The method of item 3, wherein the mutation at codon 54 encodes alanine (A), leucine (L), serine (S), threonine (T), methionine (M), or valise (V).
29. The method of item 3, wherein the mutation at codon 82 encodes threonine (T), alanine (A), phenylalanine (F), or serine (S).
30. The method of item 3, wherein the mutation at codon 84 encodes cysteine (C), alanine (A), or valine (V).
31. The method of item 3, wherein the mutation at codon 88 encodes serine (S)) or threonine (T).
32. The method of item 3, wherein the mutation at codon 90 encodes methionine (M).
33. A method for determining whether an HIV-1 is resistant to a PI, comprising detecting whether a mutation in at least one of codon 418 or 482 is present in a gene encoding gag of the HIV-1, wherein the presence of the mutation correlates with resistance to a PI, thereby determining whether the HIV-1 is resistant to the PI.
34. The method of item 33, further comprising detecting whether a mutation in at least one of codon 431, 437, 449, or 453 is present in a gene encoding gag of the HIV-1 in combination with the mutation in at least one of codon 418 or 482, wherein the presence of the mutations correlates with resistance to a PI, thereby determining whether the HIV-1 is resistant to the PI.
35. The method of item 33 or 34, wherein the mutation at codon 418 encodes glutamic acid (E) or arginine (R).
36. The method of item 33 or 34, wherein the mutation at codon 482 encodes glycine (G).
37. The method of item 34, wherein the mutation at codon 431 encodes valine (V).
38. The method of item 34, wherein the mutation at codon 437 encodes valine (V).
39. The method of item 34, wherein the mutation at codon 449 encodes isoleucine (I) or proline (P).
40. The method of item 34, wherein the mutation at codon 453 encodes leucine (L).
41. The method of item 1, 2, 3, 33, or 34, wherein the PI is amprenavir (AMP), indinavir (IDV), nelfinavir (NFV), ritonavir (RTV), saquinavir (SQV), lopinavir (LPV) or atazanavir (ATV).
42. The method of item 41, wherein the PI is AMP.
43. The method of item 41, wherein the PI is BDV.
44. The method of item 41, wherein the PI is NFV.
45. The method of item 41, wherein the PI is RTV.
46. The method of item 41, wherein the PI is SQV.
47. The method of item 41, wherein the PI is LPV.
48. The method of item 41, wherein the PI is ATV.

## Claims

1. A method for determining whether an HIV-1 is resistant to a protease inhibitor (PI), comprising detecting whether a mutation in codon 74 in combination with mutations in codons 10, 13, 36 and 71 is present in a gene encoding protease of the HIV-1, wherein the presence of a mutation in codon 74 in combination with a mutation in at least one of codons 10, 13, 36 and 71 correlates with resistance to a PI, thereby determining whether the HIV-1 is resistant to the PI, wherein the mutation at codon 74 encodes lysine, serine or proline, the mutation at 10 encodes isoleucine (I) or valine (V), the mutation at codon 13 encodes valine, the mutation at 36 encodes isoleucine or valine, the mutation at codon 71 encodes isoleucine, threonine, or valine, and wherein the codon numbering is based on the protease coding sequence present in NL4-3 HIV.

2. The method of claim 1, wherein the method further comprises detecting whether mutations at codon 437 and codon 418 are present in a gene encoding gag of the HIV-1, and wherein the mutation at codon 437 encodes valine and the mutation at codon 418 encodes glutamic acid or arginine.

3. The method of claim 1, further comprising detecting whether a mutation in at least one of codons 19, 20, 37, 54, 73, 82, 88, or 93 is present in the gene encoding protease of the HIV-1, wherein the presence of the mutation correlates with resistance to a PI, thereby determining whether the HIV-1 is resistant to the PI.

4. The method of claim 1, further comprising detecting whether a mutation in at least one of codons 23, 24, 30, 32, 46, 47, 48, 50, 54, 82, 84, 88, or 90 is present in the gene encoding protease of the HIV-1, wherein the presence of the mutation correlates with resistance to a PI, thereby determining whether the HIV-1 is resistant to the PI.

5. The method of claim 1, 3, or 4, further comprising the detection of at least one of a mutation in codons 22, 69, or 83, wherein the mutation at codon 22 encodes valine, the mutation at codon 69 encodes arginine, and the mutation at codon 83 encodes aspartic acid

6. The method of claim 3, wherein the mutation at codon 93 encodes leucine (L); or
the mutation at codon 19 encodes isoleucine (I) or valine (V); or
the mutation at codon 20 encodes isoleucine (I), methionine (M), or threonine (T); or
the mutation at codon 37 encodes aspartic acid (D); or
the mutation at codon 54 encodes valine (V); or
mutation at codon 73 encodes serine (S); or
the mutation at codon 82 encodes isoleucine (I); or
the mutation at codon 88 encodes aspartic acid (D).

7. The method of claim 4, wherein the mutation at codon 23 encodes isoleucine (I); or
the mutation at codon 24 encodes isoleucine (I) or valine (V); or
the mutation at codon 30 encodes asparagine (N); or
the mutation at codon 32 encodes alanine (A) or isoleucine (I); or
the mutation at codon 46 encodes phenylalanine (F), isoleucine (I), leucine (L), or valine (V); or
the mutation at codon 47 encodes alanine (A) or valine (V); or
the mutation at codon 48 encodes alanine (A), glutamic acid (E), leucine (L), methionine (M), or valine (V); or
the mutation at codon 50 encodes leucine (L) or valine (V); or
the mutation at codon 54 encodes alanine (A), leucine (L), serine (S), threonine (T), methionine (M), or valine (V); or
the mutation at codon 82 encodes threonine (T), alanine (A), phenylalanine (F), or serine (S); or
the mutation at codon 84 encodes cysteine (C), alanine (A), or valine (V); or
the mutation at codon 88 encodes serine (S) or threonine (T); or
the mutation at codon 90 encodes methionine (M).

8. The method of claim 1, 3, or 4, wherein the PI is amprenavir (AMP), indinavir (IDV), nelfinavir (NFV), ritonavir (RTV), saquinavir (SQV), lopinavir (LPV) or atazanavir (ATV).
